# EUROPEAN PATENT SPECIFICATION

(11) **EP 4 403 554 B1**
(45) Date of publication and mention of the grant of the patent: **24.09.2025**
(21) Application number: 23803822.8
(22) Date of filing: 09.05.2023
(51) Int. Cl.: C07D 407/12, C07D 307/91, C07D 407/14, H10K 85/60, C09D 7/63, C09K 11/06, C07D 333/76

(54) **COMPOUND, COATING COMPOSITION COMPRISING SAME, ORGANIC LIGHT-EMITTING DEVICE USING SAME, AND MANUFACTURING METHOD THEREFOR**
VERBINDUNG, BESCHICHTUNGSZUSAMMENSETZUNG DAMIT, ORGANISCHE LICHTEMITTIERENDE VORRICHTUNG DAMIT UND HERSTELLUNGSVERFAHREN DAFÜR
COMPOSÉ, COMPOSITION DE REVÊTEMENT LE COMPRENANT, DISPOSITIF ÉLECTROLUMINESCENT ORGANIQUE L'UTILISANT ET PROCÉDÉ DE FABRICATION CORRESPONDANT

(30) Priority: 10.05.2022 KR 20220057348
(43) Date of publication of application: 24.07.2024
(73) Proprietor: LG Chem, Ltd., Yeongdeungpo-gu Seoul 07336 (KR)
(72) Inventor: SEO, Minseob, Daejeon 34122 (KR); LEE, Jiyoung, Daejeon 34122 (KR); PARK, Youngju, Daejeon 34122 (KR); JANG, Songrim, Daejeon 34122 (KR); CHOI, Doowhan, Daejeon 34122 (KR); LEE, Keunsoo, Daejeon 34122 (KR); LEE, Jaechol, Daejeon 34122 (KR)
(74) Representative: Hoffmann Eitle
(86) International application number: PCT/KR2023/006302
(87) International publication number: WO 2023/219399

(56) References cited:
- WO-A1-2021/154041
- WO-A1-2021/154041
- CN-A- 106 478 566
- CN-A- 111 635 384
- KR-A- 20180 092 270
- KR-A- 20190 052 258
- KR-A- 20220 057 778

## Description

### [Technical Field]

This application claims priority to and the benefit of Korean Patent Application No. 10-2022-0057348 filed in the Korean Intellectual Property Office on May 10, 2022.

The present specification relates to a compound, a coating composition including the compound, an organic light emitting device formed using the coating composition, and a manufacturing method thereof.

### [Background Art]

An organic light emission phenomenon is one of the examples of converting an electric current into visible rays through an internal process of a specific organic molecule. The principle of the organic light emission phenomenon is as follows. When an organic material layer is disposed between an anode and a cathode and an electric current is applied between the two electrodes, electrons and holes are injected into the organic material layer from the cathode and the anode, respectively. The electrons and the holes which are injected into the organic material layer are recombined to form an exciton, and the exciton falls down again to the ground state to emit light. An organic light emitting device using this principle may be generally composed of a cathode, an anode, and an organic material layer disposed therebetween, for example, a hole injection layer, a hole transport layer, a light emitting layer, an electron injection layer and an electron transport layer. Such organic light emitting devices are known from KR 2022 0057778 A, for instance.

In order to manufacture an organic light emitting device in the related art, a deposition process has been usually used. However, there are problems in that the loss of materials occurs frequently when an organic light emitting device is manufactured by a deposition process and in that it is difficult to manufacture a device having a large area, and to solve these problems, a device using a solution process has been developed.

Therefore, there is a need for the development of a material for a solution process.

### [Detailed Description of the Invention]

### [Technical Problem]

An object of the present application is to provide a novel compound and an organic light emitting device including the same.

### [Technical Solution]

An exemplary embodiment of the present specification provides a compound represented by the following Chemical Formula 1.

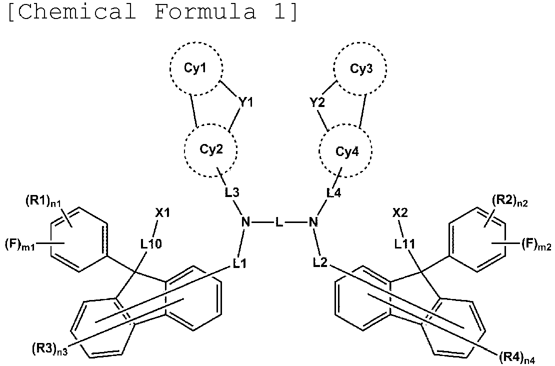

In Chemical Formula 1,
Cy1 to Cy4 are the same as or different from each other, and are each independently a substituted or unsubstituted hydrocarbon ring group,
Y1 and Y2 are the same as or different from each other, and are each independently O, S, Se or NR,
L is a substituted or unsubstituted divalent hydrocarbon ring group; or a substituted or unsubstituted divalent heterocyclic group,
L1 to L4 are the same as or different from each other, and are each independently a direct bond; a substituted or unsubstituted arylene group; or a substituted or unsubstituted heteroarylene group,
L10 and L11 are the same as or different from each other, and are each independently a substituted or unsubstituted arylene group,
X1 and X2 are the same as or different from each other, and are each independently a curable group,
R and R1 to R4 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
n1 and n2 are each an integer from 0 to 4, and when n1 and n2 are each 2 or higher, two or more substituents in the parenthesis are the same as or different from each other,
m1 and m2 are each an integer from 1 to 5, n1+m1 is 5 or lower, and n2+m2 is 5 or lower, and
n3 and n4 are each an integer from 1 to 7, and when n3 and n4 are each 2 or higher, two or more substituents in the parenthesis are the same as or different from each other.

Another exemplary embodiment of the present specification provides a coating composition including the compound.

Still another exemplary embodiment of the present specification provides an organic light emitting device including: a first electrode; a second electrode; and an organic material layer having one or more layers provided between the first electrode and the second electrode, in which one or more layers of the organic material layer include the above-described coating composition or a cured product thereof.

Yet another exemplary embodiment of the present specification provides a method for manufacturing an organic light emitting device, the method including: preparing a first electrode; forming an organic material layer having one or more layers on the first electrode; and forming a second electrode on the organic material layer, in which the forming of the organic material layer includes forming the organic material layer having one or more layers using the coating composition.

### [Advantageous Effects]

In the compound according to an exemplary embodiment of the present specification, the heteroaryl group is introduced near N to facilitate the regulation of the energy level, thereby showing an effect of improving the interfacial characteristics with other layers when applied to the organic material layer and facilitating hole migration. Further, the compound has excellent hole-mobility by including a fluoro group (-F) at a specific position.

In addition, the compound according to an exemplary embodiment of the present specification can be used as a material for an organic material layer of an organic light emitting device, and it is possible to obtain a device having low driving voltage, excellent light emitting efficiency, and/or long service life characteristics when the compound is applied to an organic light emitting device.

In addition, the compound according to an exemplary embodiment of the present specification has an advantage of forming a stable thin film undamaged from subsequent solution processes by forming fully cured thin films from a heat treatment or a light treatment.

Furthermore, since the compound according to an exemplary embodiment of the present specification shows resistance to a specific solvent after curing, a solution process can be performed when a device is manufactured, and accordingly, the device can be made to have a large area.

### [Brief Description of Drawings]

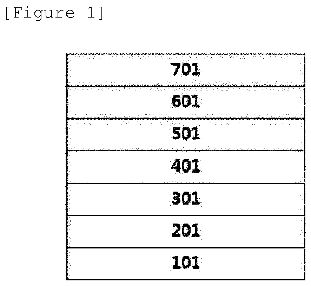

FIG. 1 is a view illustrating the structure of an organic light emitting device according to an exemplary embodiment of the present specification.
101: Substrate
201: First electrode
301: Hole injection layer
401: Hole transport layer
501: Light emitting layer
601: Electron transport and injection layer
701: Second electrode

### [Best Mode]

Hereinafter, the present specification will be described in detail.

An exemplary embodiment of the present specification provides a compound of the following Chemical Formula 1.

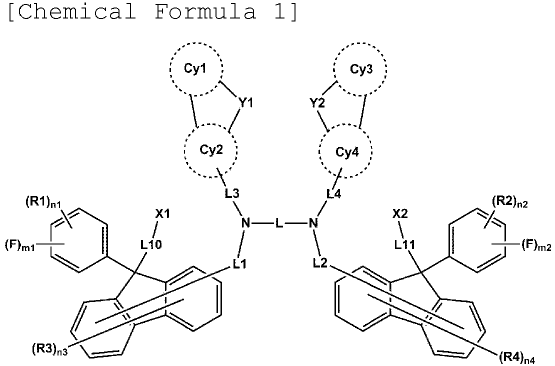

In Chemical Formula 1,
Cy1 to Cy4 are the same as or different from each other, and are each independently a substituted or unsubstituted hydrocarbon ring group,
Y1 and Y2 are the same as or different from each other, and are each independently O, S, Se or NR,
L is a substituted or unsubstituted divalent hydrocarbon ring group; or a substituted or unsubstituted divalent heterocyclic group,
L1 to L4 are the same as or different from each other, and are each independently a direct bond; a substituted or unsubstituted arylene group; or a substituted or unsubstituted heteroarylene group,
L10 and L11 are the same as or different from each other, and are each independently a substituted or unsubstituted arylene group,
X1 and X2 are the same as or different from each other, and are each independently a curable group,
R and R1 to R4 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
n1 and n2 are each an integer from 0 to 4, and when n1 and n2 are each 2 or higher, two or more substituents in the parenthesis are the same as or different from each other,
m1 and m2 are each an integer from 1 to 5, n1+m1 is 5 or lower, and n2+m2 is 5 or lower, and
n3 and n4 are each an integer from 1 to 7, and when n3 and n4 are each 2 or higher, two or more substituents in the parenthesis are the same as or different from each other.

Since the fluorenyl group of the compound of Chemical Formula 1 includes at least one fluoro group (-F), the compound has high stability and a high highest occupied molecular orbital (HOMO) energy level value.

In addition, the compound has a high highest occupied molecular orbital (HOMO) energy level value by introducing a heteroaryl group near N.

Therefore, when the compound is introduced into a hole injection layer or a hole transport layer in an organic light emitting device, the compound exhibits an effect in which the interfacial characteristics with an adjacent layer are improved and the hole migration is facilitated. In particular, when the compound of Chemical Formula 1 is included in the hole injection layer in the organic light emitting device, the compound facilitates the injection of holes from the hole injection layer to the hole transport layer, thereby greatly affecting the service life of the device.

Furthermore, since the compound has excellent solubility, there is an advantage in that various solvents can be selected when the coating composition is prepared.

Further, the compound has an advantage of forming a stable thin film undamaged from subsequent solution processes by forming fully cured thin films from a heat treatment or a light treatment.

In addition, since the compound shows resistance to a specific solvent after curing, a solution process can be performed when a device is manufactured, and accordingly, the device can be made to have a large area.

In the present specification, "energy level" means a size of energy. Therefore, the energy level is interpreted to mean the absolute value of the corresponding energy value. For example, a deep energy level means that the absolute value increases in the negative direction from the vacuum level.
When one member (layer) is disposed "on" another member (layer) in the present specification, this includes not only a case where the one member (layer) is brought into contact with another member, but also a case where still another member (layer) is present between the two members (layers).

When one part "includes" one constituent element in the present specification, unless otherwise specifically described, this does not mean that another constituent element is excluded, but means that another constituent element may be further included.

Hereinafter, the substituent of the present specification will be described in detail.

In the present specification, the "curable group" may mean a reactive substituent which cross-links compounds by being exposed to heat and/or light. The cross-linkage may be produced while radicals produced by decomposing carbon-carbon multiple bonds or cyclic structures by means of a heat treatment or light irradiation are linked to each other.

In the present specification, "Cₓ₁₋ₓ₂" means "carbon number x1 to x2".

In the present specification, **-------** means a moiety bonded to another substituent or a bonding portion.

In the present specification, an "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

In the present specification, in a ring formed by bonding adjacent groups, the "ring" means a substituted or unsubstituted hydrocarbon ring; or a substituted or unsubstituted hetero ring.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, the term "substituted or unsubstituted" means being substituted with one or two or more substituents selected from the group consisting of deuterium; a halogen group; an alkyl group; a cycloalkyl group; an alkoxy group; an aryloxy group; an amine group; an aryl group; and a heteroaryl group, being substituted with a substituent to which two or more substituents among the exemplified substituents are linked, or having no substituent. For example, "the substituent to which two or more substituents are linked" may be a biphenyl group. That is, the biphenyl group may also be an aryl group, and may be interpreted as a substituent to which two phenyl groups are linked.

Examples of the substituents will be described below, but are not limited thereto.

In the present specification, examples of a halogen group include fluorine (F), chlorine (Cl), bromine (Br) or iodine (I).

In the present specification, an alkyl group may be straight-chained or branched, and the number of carbon atoms thereof is not particularly limited, but is preferably 1 to 60. According to an exemplary embodiment, the number of carbon atoms of the alkyl group is 1 to 30. Specific examples of the alkyl group include a methyl group, an ethyl group, a propyl group, an isopropyl group, a butyl group, an isobutyl group, a tert-butyl group, a pentyl group, a hexyl group, a heptyl group, an octyl group, and the like, but are not limited thereto.

In the present specification, an alkylene group means a group having two bonding positions in an alkyl group, that is, a divalent group. The above-described description on the alkyl group may be applied to the alkylene group, except for a divalent alkylene group.

In the present specification, the number of carbon atoms of the cycloalkyl group is not particularly limited, but is preferably 3 to 60. According to an exemplary embodiment, the number of carbon atoms of the cycloalkyl group is 3 to 30. Specific examples of the cycloalkyl group include a cyclopropyl group, a cyclobutyl group, a cyclopentyl group, a cyclohexyl group, a cycloheptyl group, a cyclooctyl group, and the like, but are not limited thereto.

In the present specification, an alkoxy group may be straight-chained, branched, or cyclic. The number of carbon atoms of the alkoxy group is not particularly limited, but is preferably 1 to 30. Specific examples of the alkoxy group include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, and the like, but are not limited thereto.

In the present specification, an aryl group is not particularly limited, but may have preferably 6 to 60 carbon atoms, and may be a monocyclic aryl group or a polycyclic aryl group. According to an exemplary embodiment, the number of carbon atoms of the aryl group is 6 to 30. According to an exemplary embodiment, the number of carbon atoms of the aryl group is 6 to 20. Specific examples of the monocyclic aryl group include a phenyl group, a biphenyl group, a terphenyl group, and the like, but are not limited thereto. Examples of the polycyclic aryl group include a naphthyl group, an anthracenyl group, a phenanthrenyl group, a pyrenyl group, a perylenyl group, a triphenylenyl group, a chrysenyl group, a fluorenyl group, and the like, but are not limited thereto.

In the present specification, a fluorenyl group may be substituted, and two substituents may be bonded to each other to form a spiro structure.

When the fluorenyl group is substituted, the substituent may be 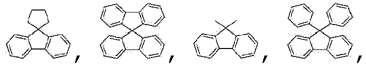 and the like, and the exemplified structure may be substituted with an additional substituent. However, the structure is not limited thereto.

In the present specification, a heterocyclic group is an aromatic group, an aliphatic group, including one or more of N, O, P and S as a heteroatom, or a fused ring group of the aromatic group and the aliphatic group. The number of carbon atoms of the heterocyclic group is not particularly limited, but may be 2 to 60.

In the present invention, a heteroaryl group is an aromatic ring group including one or more of N, O, P, and S as a heteroatom, and the number of carbon atoms thereof is not particularly limited, but may be 2 to 60. According to an exemplary embodiment, the number of carbon atoms of the heteroaryl group is 2 to 30. Examples of the heteroaryl group include a pyridine group, a pyrrole group, a pyrimidine group, a pyridazine group, a furan group, a thiophene group, a benzothiophene group, a benzofuran group, a dibenzothiophene group, a dibenzofuran group, a carbazole group, and the like, but are not limited thereto.

In the present specification, the hydrocarbon ring group may be an aromatic ring, an aliphatic ring or a ring in which an aromatic ring and an aliphatic ring are fused.

In the present specification, the above-described description on the aryl group may be applied to an aromatic ring.

In the present specification, the above-described description on the cycloalkyl group may be applied to an aliphatic ring.

In the present specification, an arylene group means a group having two bonding positions in an aryl group, that is, a divalent group. The above-described description on the aryl group may be applied to the arylene group, except for a divalent arylene group.

In the present specification, a heteroarylene group means a group having two bonding positions in a heteroaryl group, that is, a divalent group. The above-described description on the heteroaryl group may be applied to the heteroarylene group, except for a divalent heteroarylene group.

In an exemplary embodiment of the present specification, F in Chemical Formula 1 means fluorine.

In an exemplary embodiment of the present specification, X1 and X2 are the same as or different from each other, and are each independently a curable group.

In an exemplary embodiment of the present specification, the curable group is any one of the following structures. 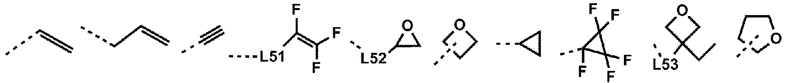 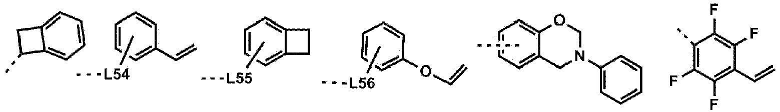 in the structures,
L51 to L56 are the same as or different from each other, and are each independently any one or two or more linked groups selected from the group consisting of a direct bond; -O-; a substituted or unsubstituted alkylene group; and a substituted or unsubstituted arylene group, and
------ is a moiety bonded to Chemical Formula 1.

In an exemplary embodiment of the present specification, X1 and X2 are the same as or different from each other, and are each independently any one of the following structures.

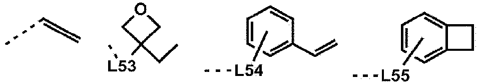

In the structures, ------ and L53 to L55 are as described above.

In an exemplary embodiment of the present specification, X1 and X2 are the same as or different from each other, and are each independently any one of the following structures.

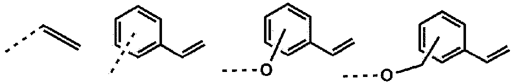

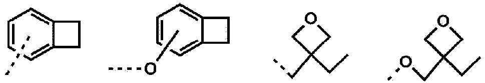

In an exemplary embodiment of the present specification, Chemical Formula 1 is the following Chemical Formula 2. 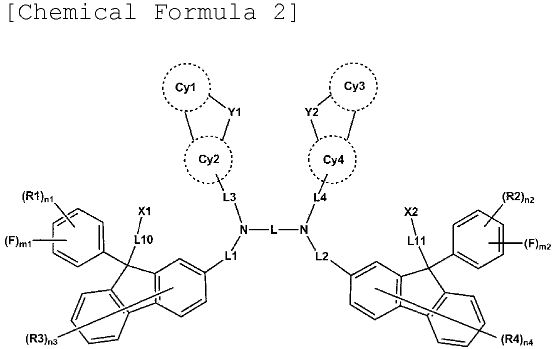 In Chemical Formula 2,
the definitions of Cy1 to Cy4, Y1, Y2, L, L1 to L4, L10, L11, X1, X2, R1 to R4, n1 to n4, m1 and m2 are the same as those in Chemical Formula 1.

In an exemplary embodiment of the present specification, L10 and L11 are the same as or different from each other, and are each independently a substituted or unsubstituted C₆₋₆₀ arylene group.

In an exemplary embodiment of the present specification, L10 and L11 are the same as or different from each other, and are each independently a substituted or unsubstituted C₆₋₃₀ arylene group.

In an exemplary embodiment of the present specification, L10 and L11 are the same as or different from each other, and are each independently a substituted or unsubstituted phenylene group; a substituted or unsubstituted biphenylene group; a substituted or unsubstituted terphenylene group; or a substituted or unsubstituted naphthylene group.

In an exemplary embodiment of the present specification, L10 and L11 are the same as or different from each other, and are each independently a substituted or unsubstituted phenylene group.

In an exemplary embodiment of the present specification, Chemical Formula 1 is the following Chemical Formula 2-1.

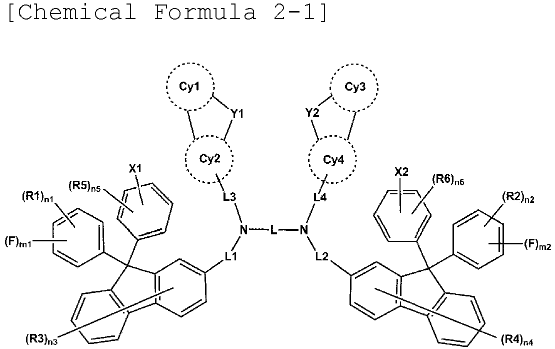

In Chemical Formula 2-1,
the definitions of Cy1 to Cy4, Y1, Y2, L, L1 to L4, X1, X2, R1 to R4, n1 to n4, m1 and m2 are the same as those in Chemical Formula 1,
R5 and R6 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group, and
n5 and n6 are each an integer from 0 to 4, and when n5 and n6 are each 2 or higher, two or more substituents in the parenthesis are the same as or different from each other.

In an exemplary embodiment of the present specification, R5 and R6 are the same as or different from each other, and are each independently hydrogen; deuterium; a halogen group; a substituted or unsubstituted C₁₋₂₀ alkyl group; a substituted or unsubstituted C₁₋₂₀ alkoxy group; a substituted or unsubstituted C₆₋₃₀ aryl group; or a substituted or unsubstituted C₂₋₃₀ heteroaryl group.

In an exemplary embodiment of the present specification, R5 and R6 are the same as or different from each other, and are each independently hydrogen; deuterium; a halogen group; a C₁₋₂₀ alkyl group; a C₁₋₂₀ alkoxy group; a C₆₋₃₀ aryl group; or a C₂₋₃₀ heteroaryl group.

In an exemplary embodiment of the present specification, R5 and R6 are each hydrogen; or deuterium.

In an exemplary embodiment of the present specification, Cy1 to Cy4 are the same as or different from each other, and are each independently a substituted or unsubstituted aromatic ring.

In an exemplary embodiment of the present specification, Cy1 to Cy4 are the same as or different from each other, and are each independently an aromatic ring.

In an exemplary embodiment of the present specification, Cy1 to Cy4 are the same as or different from each other, and are each independently a substituted or unsubstituted benzene ring; or a substituted or unsubstituted naphthalene ring.

In an exemplary embodiment of the present specification, Cy1 to Cy4 are each a benzene ring.

In an exemplary embodiment of the present specification, Chemical Formula 1 is the following Chemical Formula 3.

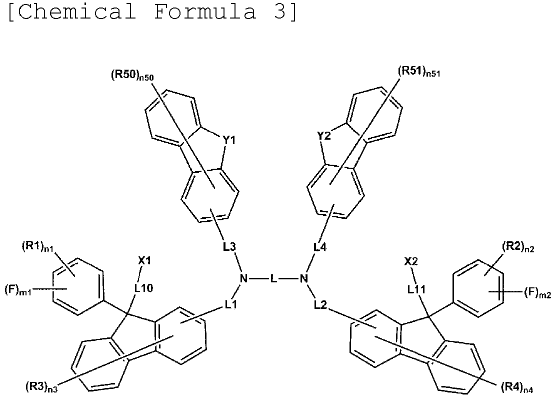

In Chemical Formula 3,
the definitions of Y1, Y2, L, L1 to L4, L10, L11, X1, X2, R1 to R4, n1 to n4, m1 and m2 are the same as those in Chemical Formula 1,
R50 and R51 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group, or are bonded to an adjacent group to form a substituted or unsubstituted ring, and
n50 and n51 are each an integer from 1 to 7, and when n50 and n51 are each 2 or higher, two or more substituents in the parenthesis are the same as or different from each other.

In an exemplary embodiment of the present specification, Chemical Formula 3 is the following Chemical Formula 3-1 or 3-2.

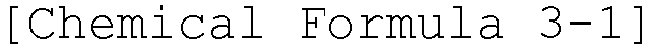

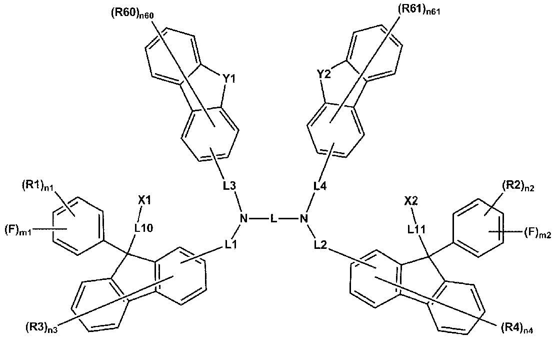

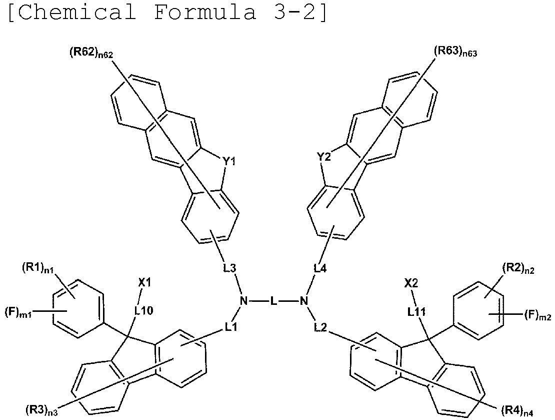

In Chemical Formulae 3-1 and 3-2,
the definitions of Y1, Y2, L, L1 to L4, L10, L11, X1, X2, R1 to R4, n1 to n4, m1 and m2 are the same as those in Chemical Formula 1,
R60 to R63 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group, and
n60 and n61 are each an integer from 1 to 7, n62 and n63 are each an integer from 1 to 9, and when n60 to n63 are each 2 or higher, two or more substituents in the parenthesis are the same as or different from each other.

In an exemplary embodiment of the present specification, Chemical Formula 3 is any one of the following Chemical Formulae 3-11 to 3-18.

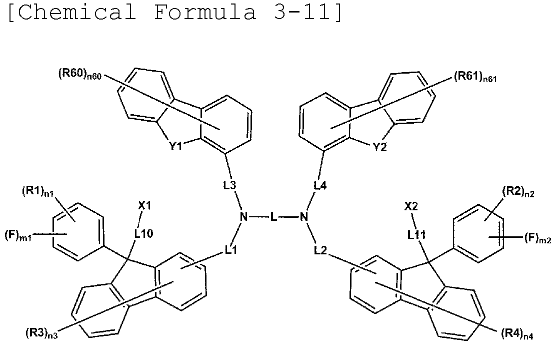

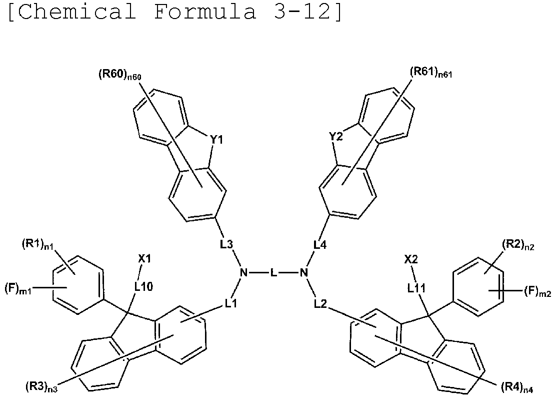

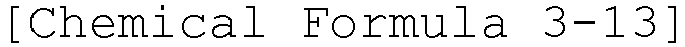

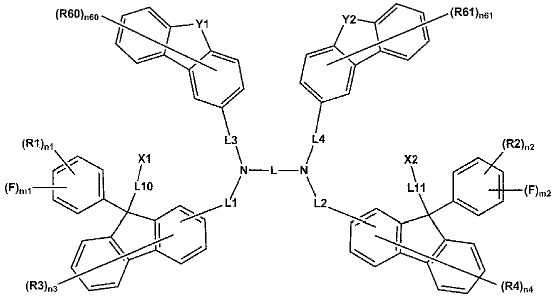

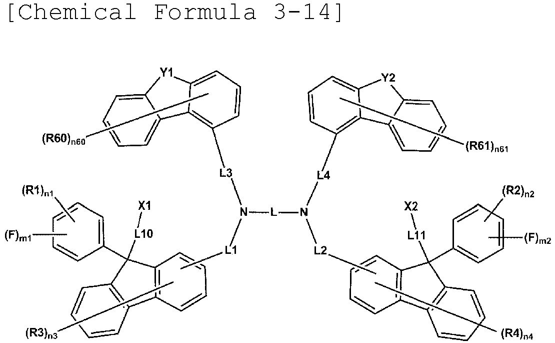

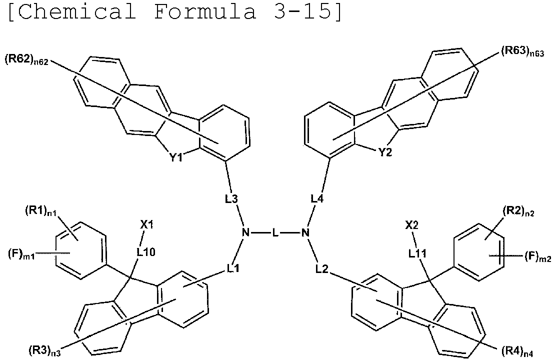

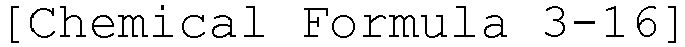

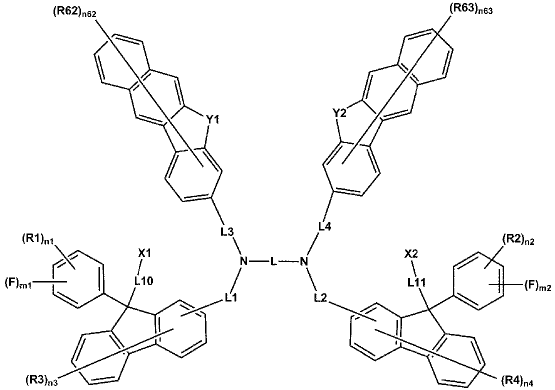

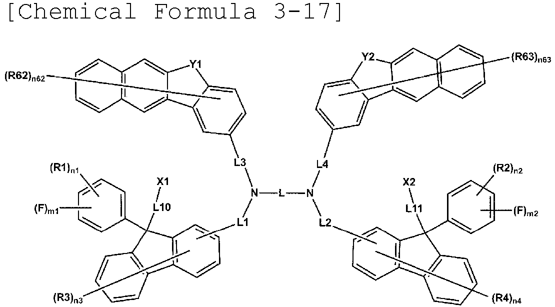

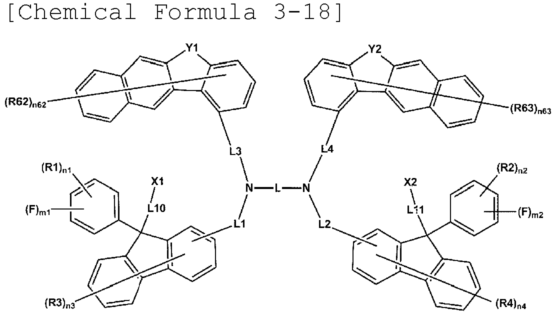

In Chemical Formulae 3-11 to 3-18,
the definitions of Y1, Y2, L, L1 to L4, L10, L11, X1, X2, R1 to R4, n1 to n4, m1 and m2 are the same as those in Chemical Formula 1,
R60 to R63 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group, and
n60 and n61 are each an integer from 1 to 7, n62 and n63 are each an integer from 1 to 9, and when n60 to n63 are each 2 or higher, two or more substituents in the parenthesis are the same as or different from each other.

In an exemplary embodiment of the present specification, R60 to R63 are each hydrogen; or deuterium.

In an exemplary embodiment of the present specification, L is a substituted or unsubstituted C₆₋₆₀ divalent hydrocarbon ring group; or a substituted or unsubstituted C₂₋₆₀ divalent heterocyclic group.

In an exemplary embodiment of the present specification, L is a substituted or unsubstituted C₆₋₃₀ divalent hydrocarbon ring group; or a substituted or unsubstituted C₂₋₃₀ divalent heterocyclic group.

According to another exemplary embodiment, L is a substituted or unsubstituted C₆₋₃₀ divalent hydrocarbon ring group.

In an exemplary embodiment of the present specification, L is a divalent hydrocarbon ring group which is unsubstituted or substituted with an alkyl group or an aryl group.

In an exemplary embodiment of the present specification, L is any one of the following structures.

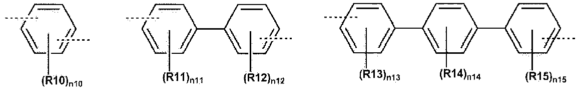

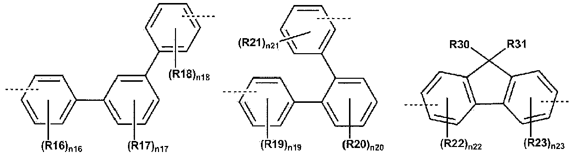

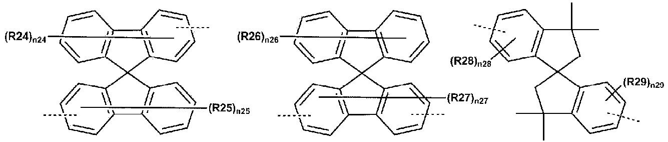

In the structures,
R10 to R31 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
n10 to n21 are each an integer from 1 to 4, n22, n23, n28 and n29 are each an integer from 1 to 3, n24 and n25 are each an integer from 1 to 7, n26 is an integer from 1 to 8, n27 is an integer from 1 to 6, and when n10 to n29 are each 2 or higher, two or more substituents in the parenthesis are the same as or different from each other, and
------ is a moiety bonded to Chemical Formula 1.

In an exemplary embodiment of the present specification, R10 to R29 are the same as or different from each other, and are each independently hydrogen; deuterium; or a substituted or unsubstituted alkyl group.

In an exemplary embodiment of the present specification, R10 to R29 are the same as or different from each other, and are each independently hydrogen; deuterium; or a substituted or unsubstituted C₁₋₂₀ alkyl group.

In an exemplary embodiment of the present specification, R10 to R29 are the same as or different from each other, and are each independently hydrogen; deuterium; or a C₁₋₂₀ alkyl group.

In an exemplary embodiment of the present specification, R30 and R31 are the same as or different from each other, and are each independently a substituted or unsubstituted alkyl group.

In an exemplary embodiment of the present specification, R30 and R31 are the same as or different from each other, and are each independently a substituted or unsubstituted C₁₋₂₀ alkyl group.

In an exemplary embodiment of the present specification, R30 and R31 are the same as or different from each other, and are each independently a C₁₋₂₀ alkyl group.

In an exemplary embodiment of the present specification, L1 to L4 are the same as or different from each other, and are each independently a direct bond; a substituted or unsubstituted C₆₋₆₀ arylene group; or a substituted or unsubstituted C₂₋₆₀ heteroarylene group.

In an exemplary embodiment of the present specification, L1 to L4 are the same as or different from each other, and are each independently a direct bond; or a substituted or unsubstituted C₆₋₃₀ arylene group.

In an exemplary embodiment of the present specification, L1 to L4 are the same as or different from each other, and are each independently a direct bond; a C₆₋₃₀ arylene group which is unsubstituted or substituted with a halogen group or an alkyl group.

In an exemplary embodiment of the present specification, L1 to L4 are the same as or different from each other, and are each independently a direct bond; a substituted or unsubstituted phenylene group; a substituted or unsubstituted biphenylene group; a substituted or unsubstituted terphenylene group; or a substituted or unsubstituted naphthylene group.

In an exemplary embodiment of the present specification, L1 to L4 are the same as or different from each other, and are each independently a direct bond; a substituted or unsubstituted phenylene group; or a substituted or unsubstituted biphenylene group.

In an exemplary embodiment of the present specification, L1 to L4 are the same as or different from each other, and are each independently a direct bond; a phenylene group which is unsubstituted or substituted with deuterium, a halogen group or an alkyl group; or a biphenylene group which is unsubstituted or substituted with deuterium, a halogen group or an alkyl group.

In an exemplary embodiment of the present specification, L1 to L4 are the same as or different from each other, and are each independently a direct bond; a phenylene group which is unsubstituted or substituted with deuterium, F or a C₁₋₂₀ alkyl group; or a biphenylene group which is unsubstituted or substituted with deuterium, F or a C₁₋₂₀ alkyl group.

In an exemplary embodiment of the present specification, R1 to R4 are the same as or different from each other, and are each independently hydrogen; deuterium; a halogen group; a substituted or un substituted C₁₋₂₀ alkyl group; a substituted or un substituted C₁₋₂₀ alkoxy group; a substituted or un substituted C₆₋₃₀ aryl group; or a substituted or un substituted C₂₋₃₀ heteroaryl group.

In an exemplary embodiment of the present specification, R1 to R4 are the same as or different from each other, and are each independently hydrogen; deuterium; or a substituted or unsubstituted C₁₋₃₀ alkyl group.

In an exemplary embodiment of the present specification, R1 to R4 are the same as or different from each other, and are each independently hydrogen; deuterium; or an alkyl group.

In an exemplary embodiment of the present specification, Y1 and Y2 are the same as or different from each other, and are each independently O or S.

In an exemplary embodiment of the present specification, m1 and m2 are each an integer from 1 to 5.

In an exemplary embodiment of the present specification, m1 is 1.

In an exemplary embodiment of the present specification, m1 is 2.

In an exemplary embodiment of the present specification, m1 is 3.

In an exemplary embodiment of the present specification, m1 is 4.

In an exemplary embodiment of the present specification, m1 is 5.

In an exemplary embodiment of the present specification, m2 is 1.

In an exemplary embodiment of the present specification, m2 is 2.

In an exemplary embodiment of the present specification, m2 is 3.

In an exemplary embodiment of the present specification, m2 is 4.

In an exemplary embodiment of the present specification, m2 is 5.

In an exemplary embodiment of the present specification, the compound of Chemical Formula 1 is any one of the following structures.

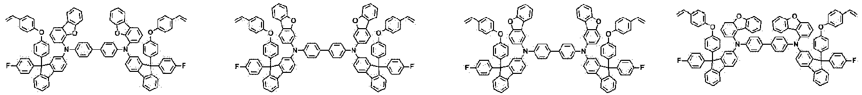

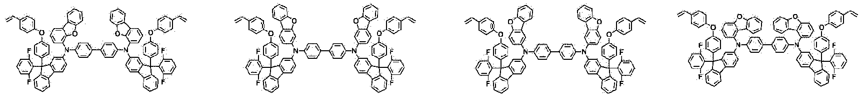

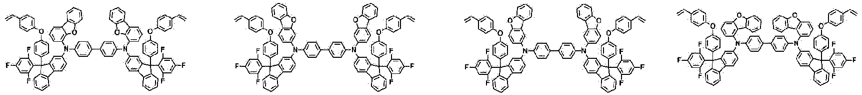

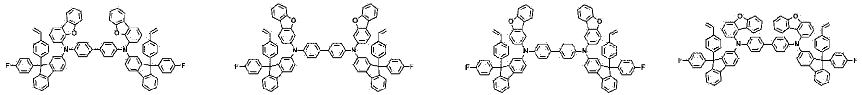

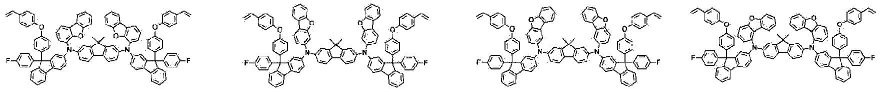

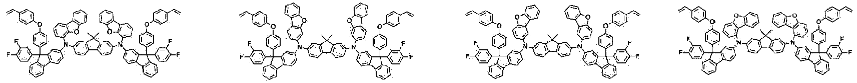

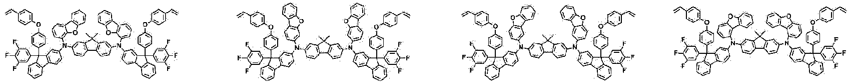

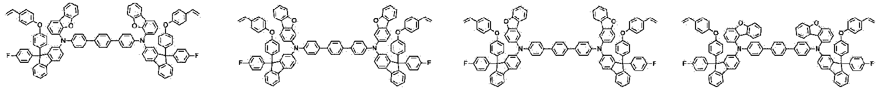

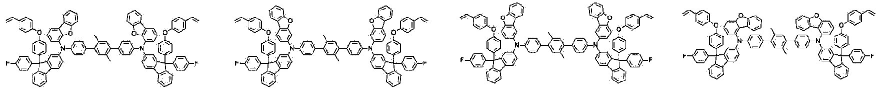

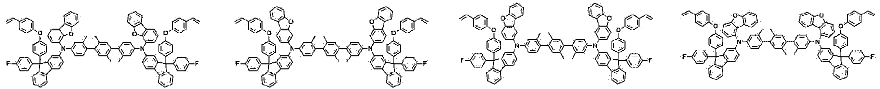

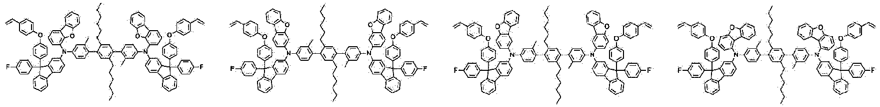

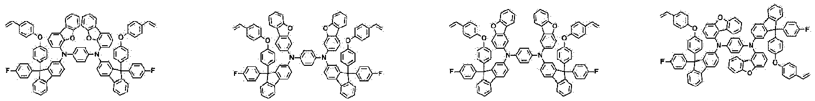

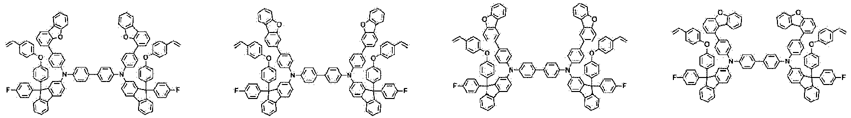

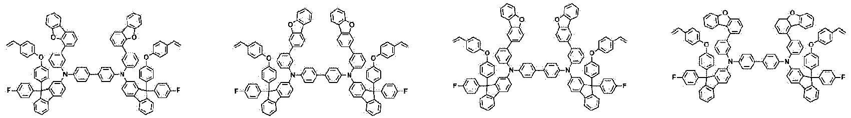

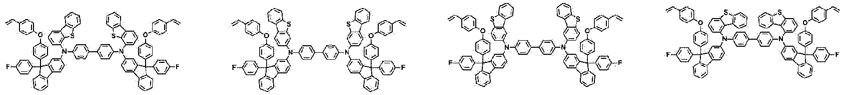

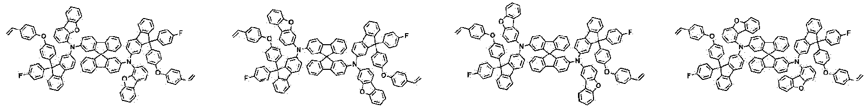

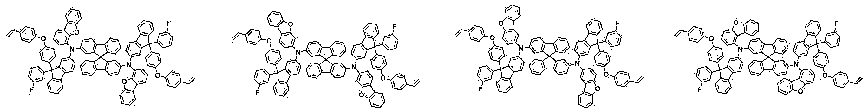

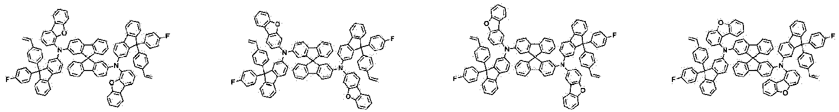

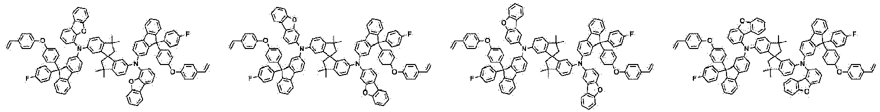

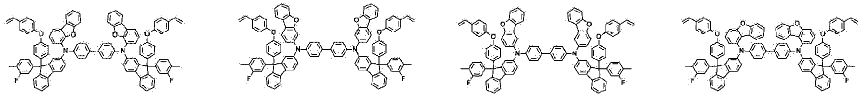

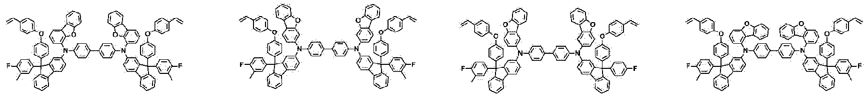

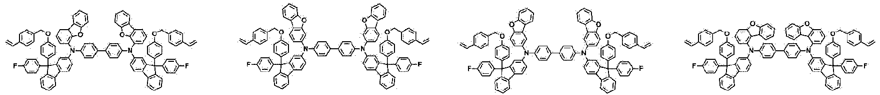

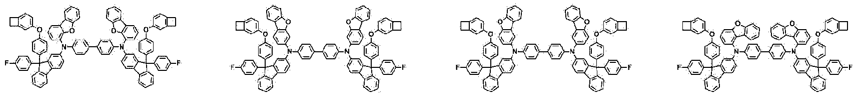

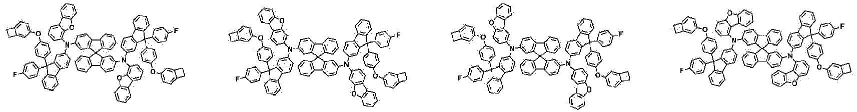

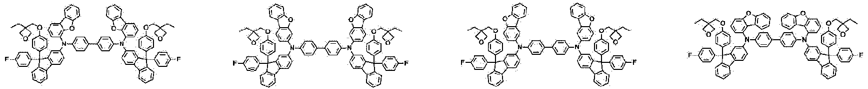

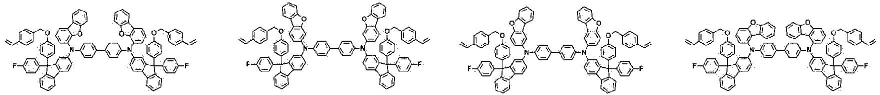

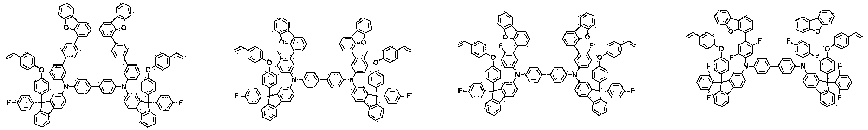

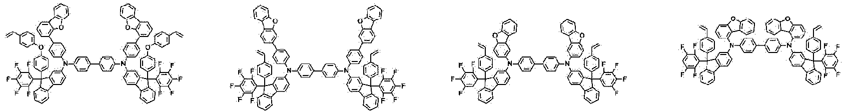

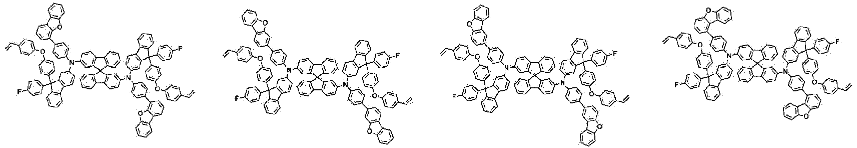

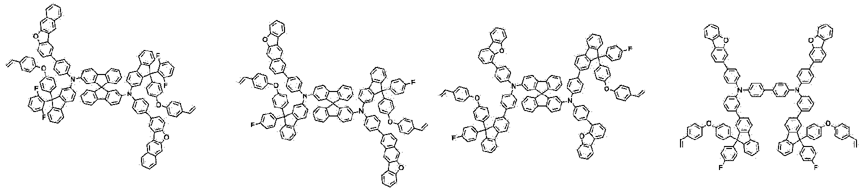

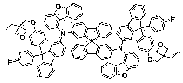

In an exemplary embodiment of the present specification, the compound of Chemical Formula 1 may be manufactured as a core structure as in the following Reaction Scheme. In this case, the substituent may be bonded by a method known in the art, and the type and position of the substituent or the number of substituents may be changed according to the technology known in the art.

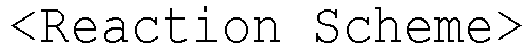

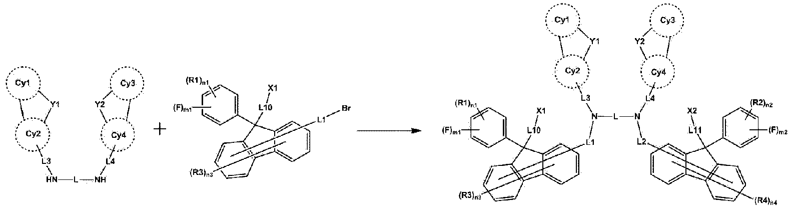

In Reaction Scheme, Cy1 to Cy4, Y1, Y2, L, L1 to L4, L10, L11, X1, X2, R1 to R4, n1 to n4, m1 and m2 are the same as those defined in Chemical Formula 1.

An exemplary embodiment of the present specification provides a coating composition including the above-described compound of Chemical Formula 1.

In an exemplary embodiment of the present specification, the coating composition further includes a solvent. In an exemplary embodiment of the present specification, the coating composition includes the compound of Chemical Formula 1 and a solvent.

In an exemplary embodiment of the present specification, the coating composition may be in a liquid phase. The "liquid phase" means that the composition is in a liquid state at room temperature under atmospheric pressure.

In an exemplary embodiment of the present specification, when the coating composition is applied to an organic material layer, a solvent that does not dissolve a material in a lower layer is used. Accordingly, there is an advantage in that the organic material layer can be introduced by the solution process.

In an exemplary embodiment of the present specification, since the compound includes a curable group, the resistance to solvent is improved during the heat treatment after coating. That is, the compound is crosslinked after coating, and thus is not dissolved in a specific solvent.

For example, even though a coating composition is prepared using a solvent that dissolves the compound and a layer is manufactured by a solution process, the layer may have resistance to the same solvent when cured through heat treatment.

Therefore, when an organic material layer is formed using the coating composition and then subjected to a curing process, a solution process can be performed when another organic material layer is applied.

In an exemplary embodiment of the present specification, the curing process may be photocuring or thermal curing. For example, after an organic material layer is formed using the coating composition, the upper layer can be introduced by a solution process because the organic material layer is cured by a heat treatment or light treatment process.

For example, when the coating composition is applied to a hole injection layer, there is an advantage in that when an upper layer (a hole transport layer and the like) is manufactured, the upper layer can be introduced by a solution process using a specific solvent to which the cured coating composition shows resistance.

In an exemplary embodiment of the present specification, the solvent included in the coating composition is a solvent that dissolves the compound. Examples of the solvent include: a chlorine-based solvent such as chloroform, methylene chloride, 1,2-dichloroethane, 1,1,2-trichloroethane, chlorobenzene, and o-dichlorobenzene; an ether-based solvent such as tetrahydrofuran and dioxane; an aromatic hydrocarbon-based solvent such as toluene, xylene, trimethylbenzene, and mesitylene; a ketone-based solvent such as acetone, methyl ethyl ketone, and cyclohexanone; an ester-based solvent such as ethyl acetate, butyl acetate, and ethyl cellosolve acetate; a polyhydric alcohol such as ethylene glycol, ethylene glycol monobutyl ether, ethylene glycol monoethyl ether, ethylene glycol monomethyl ether, dimethoxy ethane, propylene glycol, diethoxymethane, triethylene glycol monoethyl ether, glycerin, and 1,2-hexanediol and a derivative thereof; an alcohol-based solvent such as methanol, ethanol, propanol, isopropanol, and cyclohexanol; a sulfoxide-based solvent such as dimethyl sulfoxide; an amide-based solvent such as N-methyl-2-pyrrolidone and N,N-dimethylformamide; a benzoate-based solvent such as methyl benzoate, butyl benzoate, and 3-phenoxybenzoate; and a solvent such as tetralin, but the solvent can be used as long as the solvent can dissolve or disperse the compound according to an exemplary embodiment of the present specification, and is not limited thereto.

In an exemplary embodiment of the present specification, the solvents may be used either alone or in a mixture of two or more solvents.

In an exemplary embodiment of the present specification, the coating composition does not further include a p-doping material.

In an exemplary embodiment of the present specification, the coating composition further includes a p-doping material.

In the present specification, the p-doping material means a material which allows a host material to have p-semiconductor characteristics. The p-semiconductor characteristics mean characteristics of receiving or transporting holes at the highest occupied molecular orbital (HOMO) energy level, that is, characteristics of a material having large hole conductivity.

The p-doping material may be any one of the following structures, but is not limited thereto.

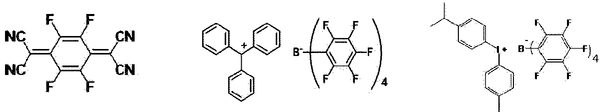

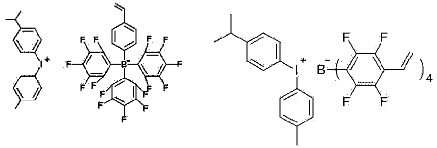

In the present specification, the p-doping material is sufficient as long as the material is a material which allows a host material to have p-semiconductor characteristics, one or two or more thereof may be used, and the type thereof is not limited.

In an exemplary embodiment of the present specification, the content of the p-doping material is 0 wt% to 500 wt% based on the compound of Chemical Formula 1. Specifically, the content of the p-doping material is 100 wt% to 400 wt% based on the compound of Chemical Formula 1.

In an exemplary embodiment of the present specification, the p-doping material is included in an amount of 0 to 50 wt% based on the total solid content of the coating composition. In an exemplary embodiment of the present specification, it is preferred that the p-doping material is included in an amount of 1 to 50 wt% based on the total solid content of the coating composition, and it is more preferred that the p-doping material is included in an amount of 10 to 30 wt% based on the total solid content of the coating composition.

In another exemplary embodiment, the coating composition further includes: a single molecule including a cross-linkable functional group by heat or light; or a single molecule including an end group capable of forming a polymer by heat.

In an exemplary embodiment of the present specification, the single molecule including a cross-linkable functional group by heat or light; or the single molecule including an end group capable of forming a polymer by heat may be a compound having a molecular weight of 3,000 g/mol or less.

In an exemplary embodiment of the present specification, the single molecule including a cross-linkable functional group by heat or light; or the single molecule including an end group capable of forming a polymer by heat may mean a single molecule in which a cross-linkable functional group by heat or light or an end group capable of forming a polymer by heat is substituted in an aryl such as phenyl, biphenyl, fluorene, and naphthalene; arylamine; or fluorene.

In an exemplary embodiment of the present specification, the coating composition has a viscosity of 2 cP to 15 cP at room temperature. Specifically, the coating composition has a viscosity of 2 cP to 10 cP. When the above viscosity is satisfied, a device is easily manufactured.

An exemplary embodiment of the present specification provides an organic light emitting device formed using the coating composition.

An exemplary embodiment of the present specification provides an organic light emitting device including: a first electrode; a second electrode; and an organic material layer having one or more layers provided between the first electrode and the second electrode, in which one or more layers of the organic material layer include the coating composition or a cured product thereof. In this case, the cured product of the coating composition is in a state in which the coating composition is cured by a heat treatment or a light treatment.

In an exemplary embodiment of the present specification, the organic material layer including the coating composition or the cured product thereof is a hole transport layer, a hole injection layer, or a layer which simultaneously transports and injects holes.

In an exemplary embodiment of the present specification, the organic material layer including the coating composition or the cured product thereof is an electron transport layer, an electron injection layer, or a layer which simultaneously transports and injects electrons.

In an exemplary embodiment of the present specification, the organic material layer including the coating composition or the cured product thereof is a light emitting layer.

In an exemplary embodiment of the present specification, the organic material layer including the coating composition or the cured product thereof is a light emitting layer, and the light emitting layer includes the compound of the present invention as a host of the light emitting layer.

In an exemplary embodiment of the present specification, the organic material layer including the coating composition or the cured product thereof is a light emitting layer, and the light emitting layer includes the compound of the present invention as a dopant of the light emitting layer.

In an exemplary embodiment of the present specification, the organic light emitting device includes one layer or two or more layers selected from the group consisting of a hole injection layer, a hole transport layer, an electron transport layer, an electron injection layer, an electron blocking layer, a hole blocking layer, a layer which simultaneously injects and transports holes, and a layer which simultaneously transports and injects electrons.

In an exemplary embodiment of the present specification, the first electrode is an anode, and the second electrode is a cathode.

According to another exemplary embodiment, the first electrode is a cathode, and the second electrode is an anode.

In another exemplary embodiment, the organic light emitting device may be a normal type organic light emitting device in which an anode, an organic material layer having one or more layers, and a cathode are sequentially stacked on a substrate.

In yet another exemplary embodiment, the organic light emitting device may be an inverted type organic light emitting device in which a cathode, an organic material layer having one or more layers, and an anode are sequentially stacked on a substrate.

The organic material layer of the organic light emitting device of the present specification may also have a single-layered structure, but may have a multi-layered structure in which two or more organic material layers are stacked. For example, the organic light emitting device of the present invention may have a structure including two or more layers selected from among a hole injection layer, a hole transport layer, a light emitting layer, an electron transport layer, an electron injection layer, a layer which simultaneously injects and transports holes, and a layer which simultaneously transports and injects electrons as an organic material layer. However, the structure of the organic light emitting device is not limited thereto, and may include a fewer number of organic layers.

For example, the structure of the organic light emitting device according to an exemplary embodiment of the present specification is exemplified in FIG. 1.

FIG. 1 exemplifies a structure of an organic light emitting device in which a first electrode 201, a hole injection layer 301, a hole transport layer 401, a light emitting layer 501, an electron transport and injection layer 601, and a second electrode 701 are sequentially stacked on a substrate 101.

FIG. 1 exemplifies an organic light emitting device, and the structure of the organic light emitting device of the present invention is not limited thereto.

When the organic light emitting device includes a plurality of organic material layers, the organic material layers may be formed of the same material or different materials.

The organic light emitting device of the present invention may be stacked as a structure in the following examples.
(1) Anode/Hole transport layer/Light emitting layer/Cathode
(2) Anode/Hole injection layer/Hole transport layer/Light emitting layer/Cathode
(3) Anode/Hole injection layer/Hole buffer layer/Hole transport layer/Light emitting layer/Cathode
(4) Anode/Hole transport layer/Light emitting layer/Electron transport layer/Cathode
(5) Anode/Hole transport layer/Light emitting layer/Electron transport layer/Electron injection layer/Cathode
(6) Anode/Hole injection layer/Hole transport layer/Light emitting layer/Electron transport layer/Cathode
(7) Anode/Hole injection layer/Hole transport layer/Light emitting layer/Electron transport layer/Electron injection layer/Cathode
(8) Anode/Hole injection layer/Hole buffer layer/Hole transport layer/Light emitting layer/Electron transport layer/Cathode
(9) Anode/Hole injection layer/Hole buffer layer/Hole transport layer/Light emitting layer/Electron transport layer/Electron injection layer/Cathode
(10) Anode/Hole transport layer/Electron blocking layer/Light emitting layer/Electron transport layer/Cathode
(11) Anode/Hole transport layer/Electron blocking layer/Light emitting layer/Electron transport layer/Electron injection layer/Cathode
(12) Anode/Hole injection layer/Hole transport layer/Electron blocking layer/Light emitting layer/Electron transport layer/Cathode
(13) Anode/Hole injection layer/Hole transport layer/Electron blocking layer/Light emitting layer/Electron transport layer/Electron injection layer/Cathode
(14) Anode/Hole transport layer/Light emitting layer/Hole blocking layer/Electron transport layer/Cathode
(15) Anode/Hole transport layer/Light emitting layer/Hole blocking layer/Electron transport layer/Electron injection layer/Cathode
(16) Anode/Hole injection layer/Hole transport layer/Light emitting layer/Hole blocking layer/Electron transport layer/Cathode
(17) Anode/Hole injection layer/Hole transport layer/Light emitting layer/Hole blocking layer/Electron transport layer/Electron injection layer/Cathode
(18) Anode/Hole injection layer/Hole transport layer/Electron blocking layer/Light emitting layer/Hole blocking layer/Electron injection and transport layer/Cathode

In the structures, the "Electron transport layer/Electron injection layer" may be replaced with "Electron transport and injection layer" or "Layer which simultaneously transports and injects electrons".

Further, in the structures, the "Hole injection layer/Hole transport layer" may be replaced with the "hole injection and transport layer" or the "layer which simultaneously transports and injects holes".

The organic light emitting device of the present specification may be manufactured by the materials and methods known in the art, except that one or more layers of the organic material layer are formed using the coating composition including the compound of Chemical Formula 1.

For example, the organic light emitting device of the present specification may be manufactured by sequentially stacking an anode, an organic material layer, and a cathode on a substrate. In this case, the organic light emitting device may be manufactured by depositing a metal or a metal oxide having conductivity, or an alloy thereof on a substrate to form an anode, forming an organic material layer including a hole injection layer, a hole transport layer, a light emitting layer, and a layer which simultaneously transports and injects electrons thereon through a solution process, a deposition process, and the like, and then depositing a material, which may be used as a cathode, thereon, by using a physical vapor deposition (PVD) method such as sputtering or e-beam evaporation. In addition to the method described above, an organic light emitting device may be made by sequentially depositing a cathode material, an organic material layer, and an anode material on a substrate.

The present specification also provides a method for manufacturing an organic light emitting device formed using the coating composition.

Specifically, an exemplary embodiment of the present specification includes: preparing a first electrode; forming an organic material layer having one or more layers on the first electrode; and forming a second electrode on the organic material layer, in which the forming of the organic material layer includes forming an organic material layer having one or more layers using the coating composition.

In an exemplary embodiment of the present specification, the forming of the organic material layer having one or more layers using the coating composition uses a spin coating method.

In another exemplary embodiment, the forming of the organic material layer having one or more layers using the coating composition uses a printing method.

In an exemplary embodiment of the present specification, examples of the printing method include inkjet printing, nozzle printing, offset printing, transfer printing or screen printing, and the like, but are not limited thereto.

A solution process is suitable for the coating composition according to an exemplary embodiment of the present specification due to the structural characteristics thereof, so that the organic material layer may be formed by a printing method, and as a result, there is an economic effect in terms of time and costs when a device is manufactured.

In an exemplary embodiment of the present specification, the forming of the organic material layer having one or more layers using the coating composition includes: coating the first electrode with the coating composition; and heat-treating or light-treating the coated coating composition.

In an exemplary embodiment of the present specification, the heat-treating of the coated coating composition may be performed by a heat treatment. The heat treatment temperature in the heat-treating of the coated coating composition is 85°C to 250°C. The heat treatment temperature may be specifically 100°C to 250°C, more specifically 150°C to 250°C.

In an exemplary embodiment of the present specification, the heat treatment time in the heat-treating of the coated coating composition may be 1 minute to 2 hours, may be 1 minute to 1 hour according to an exemplary embodiment, and may be 30 minutes to 1 hour in another exemplary embodiment.

In an exemplary embodiment of the present specification, the light-treating of the coated coating composition may be performed by UV irradiation. The light-treating of the coated coating composition may be performed for 30 minutes to 5 hours.

In an exemplary embodiment of the present specification, the coating of the first electrode with the coating composition includes coating the first electrode with the coating composition and coating another organic material layer provided on the first electrode with the coating composition.

In an exemplary embodiment of the present specification, another organic material layer means an organic material layer formed of another material without including the coating composition or the cured product thereof.

In the coating of the first electrode with the coating composition, a solvent that does not dissolve a material in the lower layer is used. For example, when the coating composition is applied to a hole transport layer, the coating composition includes a solvent that does not dissolve the material in the lower layer (first electrode, hole injection layer, and the like).

Through the heat-treating or light-treating of the coated coating composition, a plurality of the compounds included in the coating composition may form a cross-linkage, thereby providing an organic material layer including a thin-filmed structure. In this case, it is possible to prevent the organic material layer from being dissolved, morphologically affected or decomposed by a solvent when another layer is stacked on the surface of the organic material layer formed using the coating composition.

Therefore, when the organic material layer formed using the coating composition is formed by a method including the heat-treating or light-treating of the coated coating composition, resistance to a solvent is increased, so that a plurality of layers may be formed by repeatedly performing solution deposition and cross-linking methods, and stability is increased, so that the service life characteristic of the device may be increased.

In an exemplary embodiment of the present specification, as the anode material, materials having a high work function are usually preferred so as to facilitate the injection of holes into an organic material layer. Specific examples of the anode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

In an exemplary embodiment of the present specification, as the cathode material, materials having a low work function are usually preferred so as to facilitate the injection of electrons into an organic material layer. Specific examples of the cathode material include: a metal such as barium, magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

In an exemplary embodiment of the present specification, the hole injection layer is a layer which injects holes from an electrode, and a hole injection material is preferably a compound which has a capability of transporting holes, and thus has an effect of injecting holes at an anode and an excellent effect of injecting holes into a light emitting layer or a light emitting material, prevents excitons produced from the light emitting layer from moving to an electron injection layer or an electron injection material, and is also excellent in the ability to form a thin film. In addition, the highest occupied molecular orbital (HOMO) of the hole injection material is preferably a value between the work function of the anode material and the HOMO of the neighboring organic material layer. Specific examples of the hole injection material include the above-described compound of Chemical Formula 1, metal porphyrin, oligothiophene, arylamine-based organic materials, hexanitrile hexaazatriphenylene-based organic materials, quinacridone-based organic materials, perylene-based organic materials, anthraquinone, polyaniline-based and polythiophene-based electrically conductive polymers, and the like, but are not limited thereto.

In an exemplary embodiment of the present specification, the hole injection layer includes the above-described compound of the present invention. Further, in an exemplary embodiment of the present specification, the hole injection layer further includes a p-doping material. The p-doping material included in the hole injection layer is as described above in the description related to the coating composition.

In an exemplary embodiment of the present specification, the hole transport layer is a layer which accepts holes from a hole injection layer and transports the holes to a light emitting layer, and a hole transport material is suitably a material having high hole mobility which may accept holes from an anode or a hole injection layer and transfer the holes to a light emitting layer. As specific examples of the hole transport material, arylamine-based organic materials, conductive polymers, block copolymers having both conjugated portions and nonconjugated portions, and the like may be used, but the hole transport material is not limited thereto. More specifically, a compound including an arylamine group may be used for the hole transport layer.

In an exemplary embodiment of the present specification, the hole transport layer includes a compound of the following Chemical Formula HT-1.

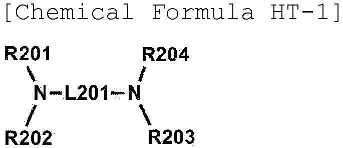

In Chemical Formula HT-1,
L201 is a substituted or unsubstituted arylene group, and
R201 to R204 are the same as or different from each other, and are each independently hydrogen; deuterium; a substituted or unsubstituted alkyl group; a substituted or unsubstituted cycloalkyl group; a substituted or unsubstituted aryl group; or a substituted or unsubstituted heterocyclic group.

In an exemplary embodiment of the present specification, L201 is a substituted or unsubstituted arylene group having 6 to 30 carbon atoms.

In an exemplary embodiment of the present specification, L201 is an arylene group.

In an exemplary embodiment of the present specification, L201 is a substituted or unsubstituted phenylene group; a substituted or unsubstituted biphenylene group; or a substituted or unsubstituted naphthylene group.

In an exemplary embodiment of the present specification, L201 is a phenylene group; a biphenylene group; or a naphthylene group.

In an exemplary embodiment of the present specification, L201 is a biphenylene group.

In an exemplary embodiment of the present specification, R201 to R204 are the same as or different from each other, and are each independently hydrogen; deuterium; a substituted or unsubstituted alkyl group having 1 to 30 carbon atoms; a substituted or unsubstituted cycloalkyl group having 3 to 30 carbon atoms; a substituted or unsubstituted aryl group having 6 to 30 carbon atoms; or a substituted or unsubstituted heterocyclic group having 2 to 30 carbon atoms.

In an exemplary embodiment of the present specification, R201 to R204 are the same as or different from each other, and are each independently a substituted or unsubstituted aryl group having 6 to 30 carbon atoms.

In an exemplary embodiment of the present specification, R201 to R204 are the same as or different from each other, and are each independently an aryl group.

In an exemplary embodiment of the present specification, R201 to R204 are the same as or different from each other, and are each independently a substituted or unsubstituted phenyl group; a substituted or unsubstituted biphenyl group; or a substituted or unsubstituted naphthyl group.

In an exemplary embodiment of the present specification, R201 to R204 are the same as or different from each other, and are each independently a phenyl group; a biphenyl group; or a naphthyl group.

In an exemplary embodiment of the present specification, Chemical Formula HT-1 is the following structure.

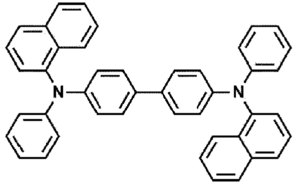

In an exemplary embodiment of the present specification, the light emitting material included in the light emitting layer is a material which may receive holes and electrons from a hole transport layer and an electron transport layer, respectively, and combine the holes and the electrons to emit light in a visible ray region, and is preferably a material having good quantum efficiency to fluorescence or phosphorescence. Specific examples thereof include: 8-hydroxy-quinoline aluminum complexes (Alq₃); carbazole-based compounds; dimerized styryl compounds; BAlq; 10-hydroxybenzoquinoline-metal compounds; benzoxazole-based, benzthiazole-based and benzimidazole-based compounds; poly(p-phenylenevinylene) (PPV)-based polymers; spiro compounds; polyfluorene, lubrene, and the like, but are not limited thereto.

In an exemplary embodiment of the present specification, the light emitting layer may include a host and a dopant. As the host, a fused aromatic ring derivative, or a hetero ring-containing compound, and the like may be used. Specifically, examples of the fused aromatic ring derivatives include anthracene derivatives, pyrene derivatives, naphthalene derivatives, pentacene derivatives, phenanthrene compounds, fluoranthene compounds, and the like, and examples of the hetero ring-containing compounds include carbazole derivatives, dibenzofuran derivatives, ladder-type furan compounds, pyrimidine derivatives, and the like, but the examples thereof are not limited thereto. More specifically, an anthracene derivative may be used as the host.

In an exemplary embodiment of the present specification, the host of the light emitting layer includes a compound of the following Chemical Formula EH-1.

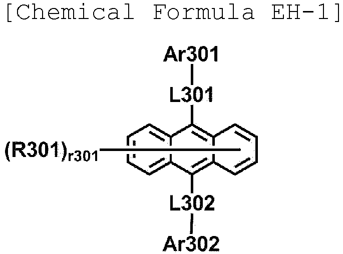

In Chemical Formula EH-1,
L301 and L302 are the same as or different from each other, and are each independently a direct bond; a substituted or unsubstituted arylene group; or a substituted or unsubstituted heterocyclic group,
Ar301 and Ar302 are the same as or different from each other, and are each independently hydrogen; deuterium; a substituted or unsubstituted aryl group; or a substituted or unsubstituted heterocyclic group,
R301 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group, and
r301 is an integer from 1 to 8, and when r301 is 2 or higher, two or more R301's are the same as or different from each other.

In an exemplary embodiment of the present specification, L301 and L302 are the same as or different from each other, and are each independently a direct bond; a substituted or unsubstituted monocyclic arylene group; or a substituted or unsubstituted polycyclic arylene group.

In an exemplary embodiment of the present specification, L301 and L302 are the same as or different from each other, and are each independently a direct bond; a substituted or unsubstituted phenylene group; a substituted or unsubstituted biphenylene group; or a substituted or unsubstituted naphthylene group.

In an exemplary embodiment of the present specification, L301 and L302 are each a direct bond.

In an exemplary embodiment of the present specification, Ar301 and Ar302 are the same as or different from each other, and are each independently a substituted or unsubstituted aryl group.

In an exemplary embodiment of the present specification, Ar301 and Ar302 are the same as or different from each other, and are each independently a substituted or unsubstituted monocyclic aryl group; or a substituted or unsubstituted polycyclic aryl group.

In an exemplary embodiment of the present specification, Ar301 and Ar302 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrene group; a substituted or unsubstituted triphenylene group; a substituted or unsubstituted pyrene group; or a substituted or unsubstituted fluorenyl group.

In an exemplary embodiment of the present specification, Ar301 and Ar302 are the same as or different from each other, and are each independently a substituted or unsubstituted phenyl group; or a substituted or unsubstituted naphthyl group.

In an exemplary embodiment of the present specification, Ar301 and Ar302 are each a naphthyl group.

In an exemplary embodiment of the present specification, R301 is hydrogen; or deuterium.

In an exemplary embodiment of the present specification, Chemical Formula EH-1 is any one of the following structures.

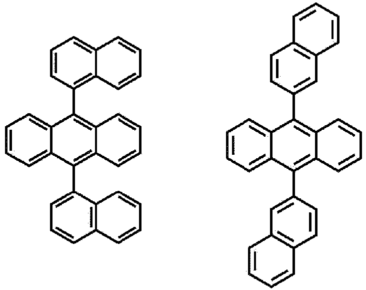

In an exemplary embodiment of the present specification, an aromatic amine derivative, a styrylamine compounds, a boron complex, a fluoranthene compound, a metal complex and the like may be used as the dopant. Specifically, the aromatic amine derivative is a fused aromatic ring derivative having a substituted or unsubstituted arylamino group, and examples thereof include a pyrene, an anthracene, a chrysene, a periflanthene, and the like, which have an arylamino group, and the styrylamine compound is a compound in which a substituted or unsubstituted arylamine is substituted with at least one arylvinyl group, and one or two or more substituents selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, examples of the metal complexes include an iridium complex, a platinum complex, and the like, but are not limited thereto. More specifically, a compound including an arylamine group may be used as the dopant.

In an exemplary embodiment of the present specification, the dopant of the light emitting layer includes a compound of the following Chemical Formula ED-1.

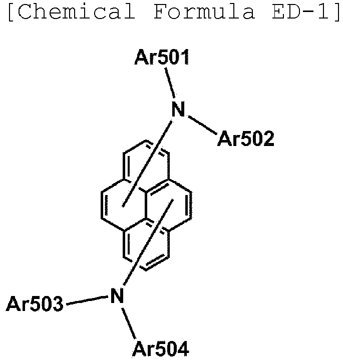

In Chemical Formula ED-1,
Ar501 to Ar504 are the same as or different from each other, and are each independently hydrogen; deuterium; a substituted or unsubstituted alkyl group; a substituted or unsubstituted aryl group; or a substituted or unsubstituted heteroaryl group.

In an exemplary embodiment of the present specification, Chemical Formula ED-1 is the following Chemical Formula ED-2.

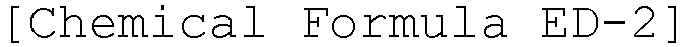

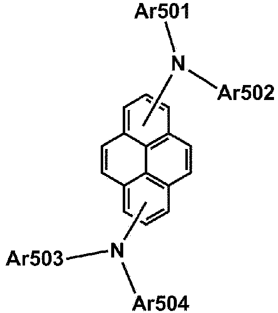

In Chemical Formula ED-2,
Ar501 to Ar504 are the same as those defined in Chemical Formula ED-1.

In an exemplary embodiment of the present specification, Ar501 to Ar504 are the same as or different from each other, and are each independently a substituted or unsubstituted aryl group.

In an exemplary embodiment of the present specification, Ar501 to Ar504 are the same as or different from each other, and are each independently a substituted or unsubstituted phenyl group; a substituted or unsubstituted biphenyl group; a substituted or unsubstituted terphenyl group; or a substituted or unsubstituted naphthyl group.

In an exemplary embodiment of the present specification, Ar501 to Ar504 are the same as or different from each other, and are each independently a phenyl group which is unsubstituted or substituted with a silyl group; a biphenyl group which is unsubstituted or substituted with a silyl group; a terphenyl group which is unsubstituted or substituted with a silyl group; or a naphthyl group which is unsubstituted or substituted with a silyl group.

In an exemplary embodiment of the present specification, Ar501 to Ar504 are the same as or different from each other, and are each independently a phenyl group which is unsubstituted or substituted with a silyl group.

In the present specification, the silyl group is a group represented by -SiRcRd, and Rc and Rd are the same as or different from each other, and are each independently hydrogen; deuterium; a substituted or unsubstituted alkyl group; a substituted or unsubstituted aryl group; or a substituted or unsubstituted heteroaryl group. The number of carbon atoms of the silyl group is not particularly limited, but is preferably 1 to 60.

In an exemplary embodiment of the present specification, Chemical Formula ED-1 is the following structure.

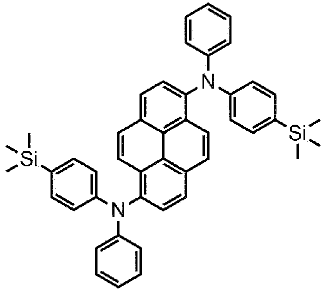

In an exemplary embodiment of the present specification, the electron transport layer is a layer that receives electrons from the electron injection layer and transports the electrons to the light emitting layer. An electron transport material is suitably a material having high electron mobility which may proficiently accept electrons from a cathode and transfer the electrons to a light emitting layer. Specific examples thereof include: Al complexes of 8-hydroxyquinoline; complexes including Alq₃; organic radical compounds; hydroxyflavone-metal complexes, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

In an exemplary embodiment of the present specification, the electron injection layer is a layer that injects electrons from the electrode. An electron injection material is preferably a compound which has a capability of transporting electrons, an effect of injecting electrons from a cathode, and an excellent effect of injecting electrons into a light emitting layer or a light emitting material, prevents excitons produced from a light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, benzoimidazole, perylenetetracarboxylic acid, phenanthroline, fluorenylidene methane, anthrone, and the like and derivatives thereof, metal complex compounds, nitrogen-containing 5-membered ring derivatives, and the like, but are not limited thereto.

In an exemplary embodiment of the present specification, examples of the metal complex compounds include 8-hydroxyquinolinato lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, bis(2-methyl-8-quinolinato) (2-naphtholato) gallium, and the like, but are not limited thereto.

In an exemplary embodiment of the present specification, the electron transport layer and electron injection layer may be formed as a layer which simultaneously transports and injects electrons, and may be expressed as an electron transport and injection layer. In this case, materials applied to the electron transport and injection layer can be both the electron transport material and electron injection material described above. For example, a benzoimidazole-based compound may be used in the electron transport and injection layer.

In an exemplary embodiment of the present specification, the electron transport and injection layer includes a compound of the following Chemical Formula ET-1.

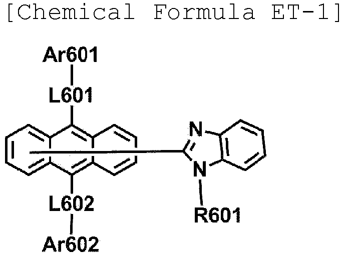

In Chemical Formula ET-1,
L601 and L602 are the same as or different from each other, and are each independently a direct bond; a substituted or unsubstituted arylene group; or a substituted or unsubstituted heterocyclic group,
Ar601 and Ar602 are the same as or different from each other, and are each independently hydrogen; deuterium; a substituted or unsubstituted aryl group; or a substituted or unsubstituted heterocyclic group, and
R601 is hydrogen; deuterium; a halogen group; a substituted or unsubstituted alkyl group; a substituted or unsubstituted cycloalkyl group; a substituted or unsubstituted aryl group; or a substituted or unsubstituted heterocyclic group.

In an exemplary embodiment of the present specification, L601 and L602 are the same as or different from each other, and are each independently a direct bond; a substituted or unsubstituted monocyclic arylene group; or a substituted or unsubstituted polycyclic arylene group.

In an exemplary embodiment of the present specification, L601 and L602 are the same as or different from each other, and are each independently a direct bond; a substituted or unsubstituted phenylene group; a substituted or unsubstituted biphenylylene group; or a substituted or unsubstituted naphthylene group.

In an exemplary embodiment of the present specification, L601 and L602 are each a direct bond.

In an exemplary embodiment of the present specification, Ar601 and Ar602 are the same as or different from each other, and are each independently a substituted or unsubstituted aryl group.

In an exemplary embodiment of the present specification, Ar601 and Ar602 are the same as or different from each other, and are each independently a substituted or unsubstituted monocyclic aryl group; or a substituted or unsubstituted polycyclic aryl group.

In an exemplary embodiment of the present specification, Ar601 and Ar602 are the same as or different from each other, and are each independently a substituted or unsubstituted phenyl group; a substituted or unsubstituted biphenyl group; a substituted or unsubstituted terphenyl group; a substituted or unsubstituted naphthyl group; a substituted or unsubstituted anthracenyl group; a substituted or unsubstituted phenanthrenyl group; a substituted or unsubstituted triphenylenyl group; a substituted or unsubstituted pyrenyl group; or a substituted or unsubstituted fluorenyl group.

In an exemplary embodiment of the present specification, Ar601 and Ar602 are the same as or different from each other, and are each independently a substituted or unsubstituted phenyl group; or a substituted or unsubstituted naphthyl group.

In an exemplary embodiment of the present specification, Ar601 and Ar602 are each a naphthyl group.

In an exemplary embodiment of the present specification, R601 is a substituted or unsubstituted aryl group.

In an exemplary embodiment of the present specification, R601 is a substituted or unsubstituted phenyl group.

In an exemplary embodiment of the present specification, Chemical Formula ET-1 is the following structure.

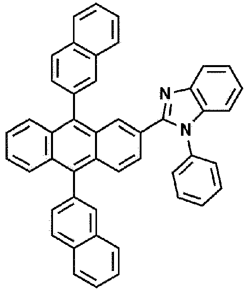

In an exemplary embodiment of the present specification, the hole blocking layer is a layer which blocks holes from reaching a cathode, and may be generally formed under the same conditions as those of the hole injection layer. Specific examples of the hole blocking material include oxadiazole derivatives or triazole derivatives, phenanthroline derivatives, BCP, aluminum complexes, and the like, but are not limited thereto.

In an exemplary embodiment of the present specification, the electron blocking layer is a layer which blocks electrons from reaching an anode, and materials known in the art may be used.

The organic light emitting device according to the present specification may be a top emission type, a bottom emission type, or a dual emission type according to the materials to be used.

### [Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

### <Preparation Examples>

### Preparation Example 1. Preparation of Compound 1

### (1) Synthesis of Intermediate 1-1

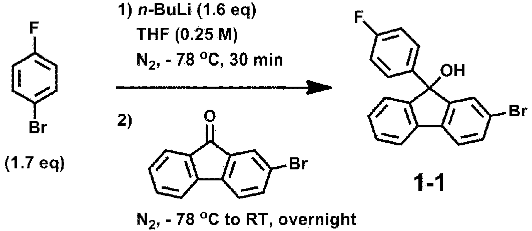

1-bromo-4-fluorobenzene (27.9 mL, 255 mmol, 1.7 eq) was put into tetrahydrofuran (THF) (500 mL). After substitution with nitrogen, the mixture was cooled to -78°C. n-Butyllithium (n-BuLi) (2.5 M in Hex) (96 mL, 240 mmol, 1.6 eq) was put into a dropping funnel, and then slowly added to the reaction mixture, and then the resulting mixture was stirred at -78°C for 30 minutes. 2-Bromofluorenone (38.9 g, 150 mmol) was added thereto. The mixture was stirred overnight while being slowly warmed to room temperature. After the reaction was terminated by adding distilled water thereto, extraction was performed with ethyl acetate and water. After an organic layer was collected, the organic layer was dried using magnesium sulfate (MgSO₄) and filtered. Intermediate 1-1 was obtained by drying the filtrate using a vacuum rotary concentrator to remove the organic solvent.

### (2) Synthesis of Intermediate 1-2

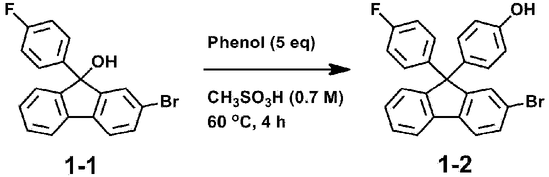

Intermediate 1-1 (53 g, 150 mmol), phenol (70.6 g, 750 mmol, 5 eq) were put into a round bottom flask (RBF). After methanesulfonic acid (CH₃SO₃H) (214 mL, 0.7 M) was added thereto, the resulting mixture was stirred at 60°C for 4 hours. After ice water was added thereto, extraction was performed with ethyl acetate and water. After an organic layer was collected, the organic layer was dried using MgSO₄ and filtered. The filtrate was dried using a vacuum rotary concentrator to remove the organic solvent. After column purification, crystallization was performed under dichloromethane/heptane conditions to obtain 40.6 g of Intermediate 1-2.

### (3) Synthesis of Intermediate 1-3

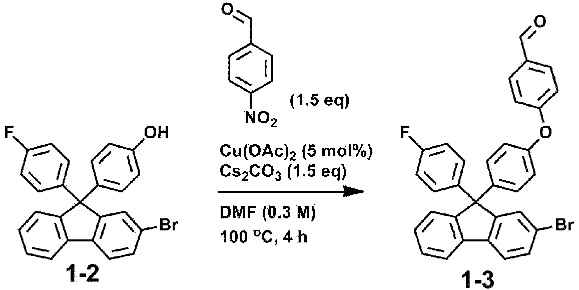

Intermediate 1-2 (40 g, 92.7 mmol), 4-nitrobenzaldehyde (21.2 g, 139 mmol, 1.5 eq), copper (II)acetate (Cu(OAc)₂) (842 mg, 4.64 mmol, 5 mol%), and cesium carbonate (Cs₂CO₃) (45.3 g, 139 mmol, 1.5 eq) were put into RBF. Dimethylformamide (DMF) (310 mL) was added thereto, and the resulting mixture was stirred at 100°C for 4 hours. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/heptane conditions to obtain 38.7 g of Intermediate 1-3.

### (4) Synthesis of Intermediate 1-4

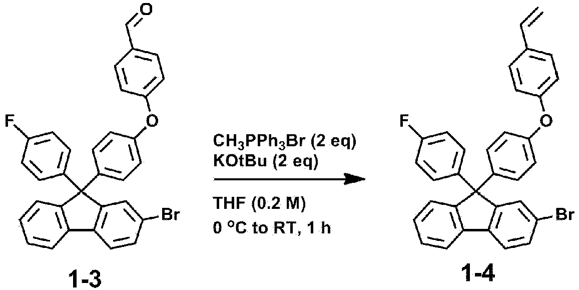

After methyltriphenylphosphonium bromide (CH₃PPh₃Br) (51.6 g, 144.6 mmol, 2 eq), potassium tertiary-butoxide (KOtBu) (16.2 g, 144.6 mmol, 2 eq), and THF (217 mL) were put into RBF, the resulting mixture was cooled to 0°C. A solution of Intermediate 1-3 (38.7 g, 72.3 mmol) in tetrahydrofuran (THF) (144 mL) was added to the reaction mixture. The resulting mixture was stirred for 1 hour while being warmed to room temperature. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under dichloromethane/ethanol (DCM/EtOH) conditions to obtain 33.7 g of Intermediate 1-4.

### (5) Synthesis of Compound 1

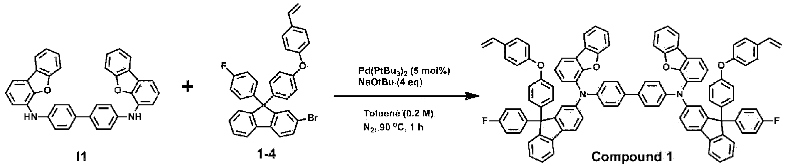

Compound I1 (1.29 g, 2.5 mmol), Intermediate 1-4 (2.93 g, 5.5 mmol), bis(tri-tert-butylphosphine)palladium(0) (Pd(PtBu₃)₂) (64 mg, 0.125 mmol, 5 mol%) and sodium tert-butoxide (NaOtBu) (961 mg, 10 mmol, 4 eq) were put into RBF. After substitution with nitrogen, toluene (12.5 mL) was added thereto, and the resulting mixture was stirred at 90°C for 1 hour. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 2.6 g of Compound 1. It was confirmed through LC-MS and NMR that Compound 1 was synthesized. MS: [M+H]⁺ = 1422
NMR measurement value of Compound 1: ¹H NMR (500 MHz, DMSO-d6) δ 8.02 (m, 2H), 7.87 - 7.70 (m, 8H), 7.47 - 7.32 (m, 8H), 7.31 - 7.15 (m, 10H), 7.05 - 6.90 (m, 8H), 6.90 - 6.85 (m, 12H), 6.77 - 6.65 (m, 8H), 6.53 (d, 4H), 6.51 (m, 2H), 6.36 (m, 2H), 5.71 (dd, 2H), 5.18 (dd, 2H)

### Preparation Example 2. Preparation of Compound 2

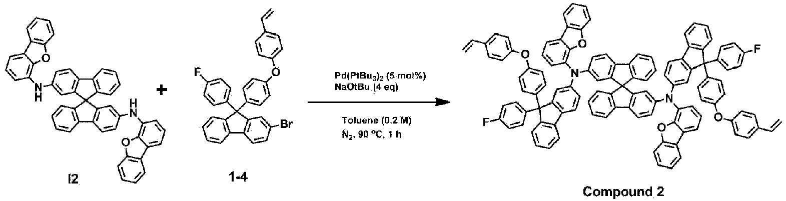

2.8 g of Compound 2 was obtained by preparation in the same manner as in Preparation Example 1, except that Compound I2 was used instead of Compound I1 in (5) of Preparation Example 1. It was confirmed through LC-MS and NMR that Compound 2 was synthesized. MS: [M+H]⁺ = 1584
NMR measurement value of Compound 2: ¹H NMR (500 MHz, DMSO-d6) δ 8.02 (m, 2H), 7.86 - 7.69 (m, 10H), 7.44 - 7.33 (m, 8H), 7.31 - 7.15 (m, 12H), 7.01 - 6.91 (m, 8H), 6.87 - 6.81 (m, 12H), 6.78 - 6.65 (m, 8H), 6.55 (d, 4H), 6.50 (m, 2H), 6.35 (m, 2H), 5.71 (dd, 2H), 5.18 (dd, 2H)

### Preparation Example 3. Preparation of Compound 3

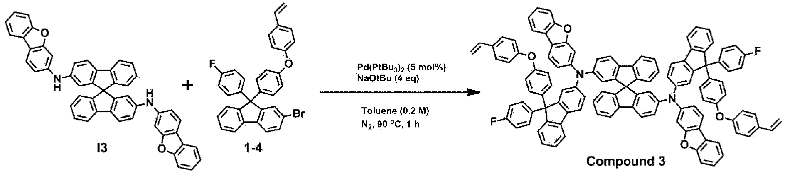

2.6 g of Compound 3 was obtained by preparation in the same manner as in Preparation Example 1, except that Compound I3 was used instead of Compound I1 in (5) of Preparation Example 1. It was confirmed through LC-MS and NMR that Compound 3 was synthesized. MS: [M+H]⁺ = 1584
NMR measurement value of Compound 3: ¹H NMR (500 MHz, DMSO-d6) δ 7.93 (m, 2H), 7.80 - 7.73 (m, 10H), 7.41 - 7.25 (m, 18H), 7.09 - 6.80 (m, 32H), 6.70 - 6.61 (m, 4H), 6.45 (m, 2H), 5.68 (dd, 2H), 5.18 (dd, 2H)

### Preparation Example 4. Preparation of Compound 4

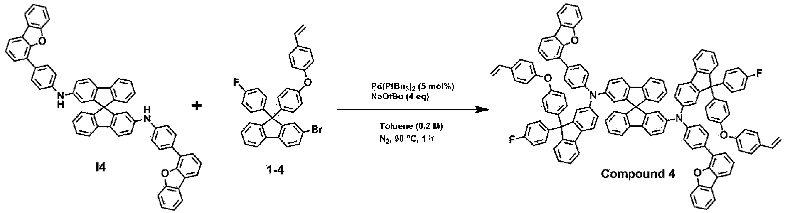

2.9 g of Compound 4 was obtained by preparation in the same manner as in Preparation Example 1, except that Compound I4 was used instead of Compound I1 in (5) of Preparation Example 1. It was confirmed through LC-MS and NMR that Compound 4 was synthesized. MS: [M+H]⁺ = 1736
NMR measurement value of Compound 4: ¹H NMR (500 MHz, DMSO-d6) δ 8.07 (m, 2H), 7.98 (m, 2H), 7.82 - 7.67 (m, 12H), 7.47 (m, 2H), 7.39 - 7.22 (m, 20H), 7.09 - 6.93 (m, 24H), 6.89 - 6.84 (m, 6H), 6.79 (m, 2H), 6.64 - 6.50 (m, 6H), 5.62 (dd, 2H), 5.12 (dd, 2H)

### Preparation Example 5. Preparation of Compound 5

### (1) Synthesis of Intermediate 5-1

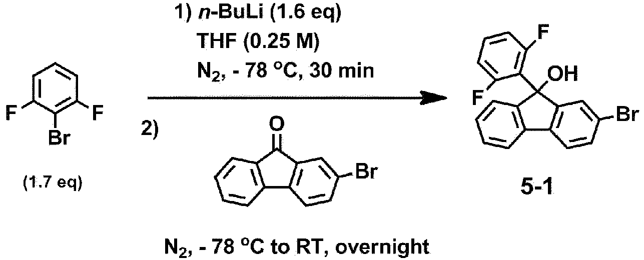

Intermediate 5-1 was prepared in the same manner as in (1) of Preparation Example 1, except that 1-bromo-2,6-difluorobenzene (29.4 mL, 255 mmol, 1.7 eq) was used instead of 1-bromo-4-fluorobenzene in (1) of Preparation Example 1.

### (2) Synthesis of Intermediate 5-2

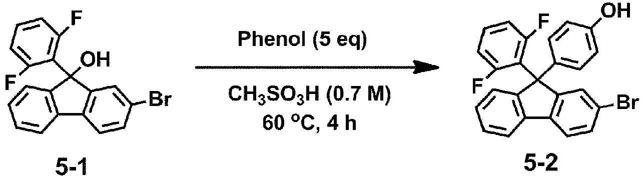

45.2 g of Intermediate 5-2 was obtained by preparation in the same manner as in (2) of Preparation Example 1, except that Intermediate 5-1 (56 g, 150 mmol) was used instead of Intermediate 1-1 in (2) of Preparation Example 1.

### (3) Synthesis of Intermediate 5-3

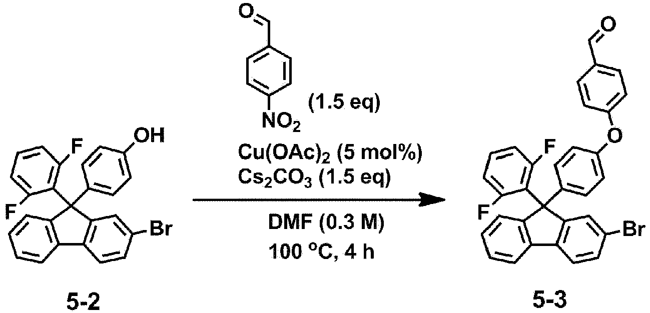

39.8 g of Intermediate 5-3 was obtained by preparation in the same manner as in (3) of Preparation Example 1, except that Intermediate 5-2 (45.2 g, 100 mmol) was used instead of Intermediate 1-2 in (3) of Preparation Example 1.

### (4) Synthesis of Intermediate 5-4

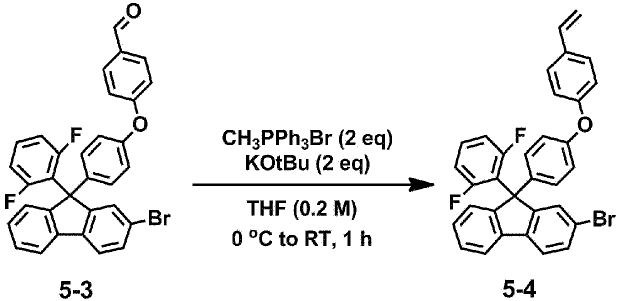

34.8 g of Intermediate 5-4 was obtained by preparation in the same manner as in (4) of Preparation Example 1, except that Intermediate 5-3 (39.8 g, 72 mmol) was used instead of Intermediate 1-3 in (4) of Preparation Example 1.

### (5) Synthesis of Compound 5

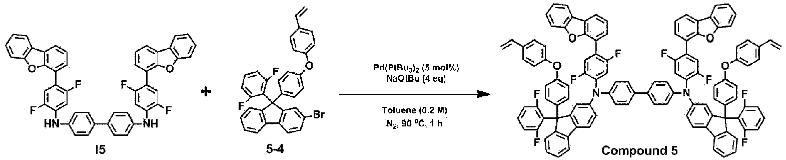

2.6 g of Compound 5 was obtained by preparation in the same manner as in (5) of Preparation Example 1, except that Compound I5 (1.85 g, 2.5 mmol) and Intermediate 5-4 (2.93 g, 5.5 mmol) were used instead of Compound I1 and Intermediate 1-4, respectively, in (5) of Preparation Example 1. It was confirmed through LC-MS and NMR that Compound 5 was synthesized. MS: [M+H]⁺ = 1682
NMR measurement value of Compound 5: ¹H NMR (500 MHz, DMSO-d6) δ 8.04 (m, 2H), 7.87 - 7.70 (m, 8H), 7.47 - 7.32 (m, 10H), 7.31 - 7.15 (m, 8H), 7.05 - 6.90 (m, 8H), 6.90 - 6.85 (m, 12H), 6.77 - 6.65 (m, 8H), 6.53 (d, 4H), 6.51 (m, 2H), 6.36 (m, 2H), 5.71 (dd, 2H), 5.18 (dd, 2H)

### Preparation Example 6. Preparation of Compound 6

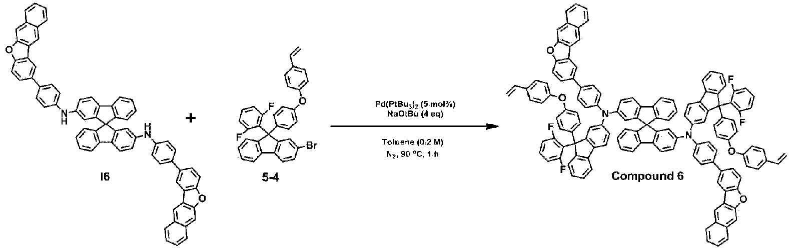

3.5 g of Compound 6 was obtained by preparation in the same manner as in (5) of Preparation Example 1, except that Compound I6 (1.85 g, 2.5 mmol) and Intermediate 5-4 (2.93 g, 5.5 mmol) were used instead of Compound I1 and Intermediate 1-4, respectively, in (5) of Preparation Example 1. It was confirmed through LC-MS and NMR that Compound 6 was synthesized. MS: [M+H]⁺ = 1873

NMR measurement value of Compound 6: ¹H NMR (500 MHz, DMSO-d6) δ 8.28 (d, 2H), 8.10 (m, 2H), 7.90 - 7.67 (m, 14H), 7.46 (m, 2H), 7.38 - 7.20 (m, 20H), 7.09 - 6.93 (m, 24H), 6.89 - 6.84 (m, 6H), 6.76 (m, 2H), 6.62 - 6.73 (m, 6H), 5.62 (dd, 2H), 5.12 (dd, 2H)

### Preparation Example 7. Preparation of Compound 7

### (1) Synthesis of Intermediate 7-1

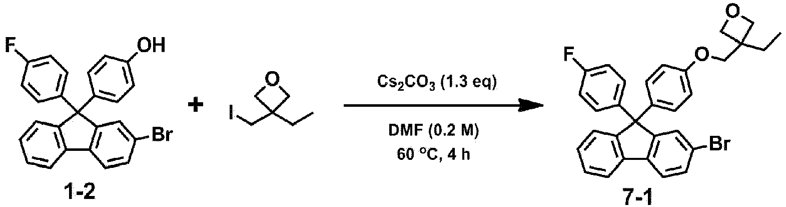

Intermediate 1-2 (8.63 g, 20 mmol) and Cs₂CO₃ (8.47 g, 26 mmol, 1.3 eq) were put into RBF. DMF (100 mL) and 3-ethyl-3-iodomethyloxetane (4 mL, 26 mmol) were added thereto, and the resulting mixture was stirred at 60°C for 4 hours. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/heptane conditions to obtain 12.3 g of Intermediate 7-1.

### (2) Synthesis of Compound 7

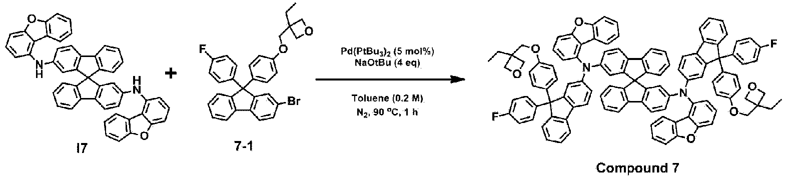

2.6 g of Compound 7 was obtained by preparation in the same manner as in (5) of Preparation Example 1, except that Compound I7 (1.7 g, 2.5 mmol) and Intermediate 7-1 (2.91 g, 5.5 mmol) were used instead of Compound I1 and Intermediate 1-4, respectively, in (5) of Preparation Example 1. It was confirmed through LC-MS and NMR that Compound 7 was synthesized. MS: [M+H]⁺ = 1576
NMR measurement value of Compound 7: ¹H NMR (500 MHz, DMSO-d6) δ 7.77 - 7.73 (m, 4H), 7.68 - 7.55 (m, 4H), 7.52 - 7.46 (m, 4H), 7.41 - 7.32 (m, 10H), 7.24 - 7.13 (m, 4H), 7.10 - 6.38 (m, 30H), 4.39 (m, 4H), 4.30 (m, 4H), 3.99 (m, 4H), 1.75 (m, 4H), 0.84 (m, 6H)

### Preparation Example 8. Preparation of Compound 8

### (1) Synthesis of Intermediate 8-1

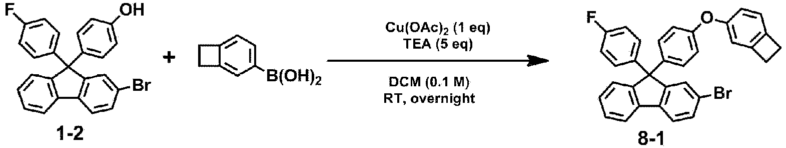

Intermediate 1-2 (39 g, 90 mmol), benzocyclobutane-4-boronic acid (20 g, 135 mmol), Cu(OAc)₂ (16.4 g, 90 mmol, 1 eq), and 4 Å molecular sieve (90 g) were put into RBF. DCM (900 mL) and triethylamine (TEA) (63 mL, 450 mmol, 5 eq) were added thereto, and the resulting mixture was stirred at room temperature overnight. Extraction was performed with ethyl acetate and water, an organic layer was collected, and then the organic layer was dried using MgSO₄ and then filtered. The filtrate was dried using a vacuum rotary concentrator to remove the organic solvent. After column purification, crystallization was performed under DCM/EtOH conditions to obtain 15 g of Intermediate 8-1.

### (2) Synthesis of Compound 8

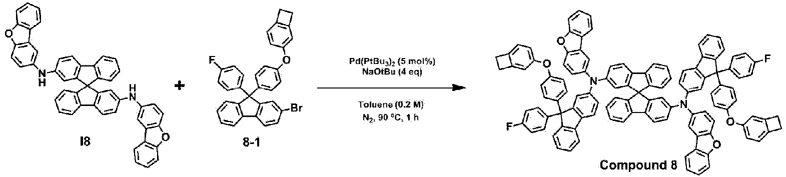

3 g of Compound 8 was obtained by preparation in the same manner as in (5) of Preparation Example 1, except that Compound I8 (1.7 g, 2.5 mmol) and Intermediate 8-1 (2.93 g, 5.5 mmol) were used instead of Compound I1 and Intermediate 1-4, respectively, in (5) of Preparation Example 1. It was confirmed through LC-MS and NMR that Compound 8 was synthesized. MS: [M+H]⁺ = 1584
NMR measurement value of Compound 8: ¹H NMR (500 MHz, DMSO-d6) δ 7.93 (m, 2H), 7.78 - 7.72 (m, 10H), 7.39 - 7.24 (m, 14H), 7.09 - 6.93 (m, 24H), 6.85 (m, 2H), 6.78 - 6.65 (m, 8H), 6.61 (d, 2H), 6.44 (m, 2H), 3.04 - 2.97 (m, 8H)

### Preparation Example 9. Preparation of Compound 9

### (1) Synthesis of Intermediate 9-1

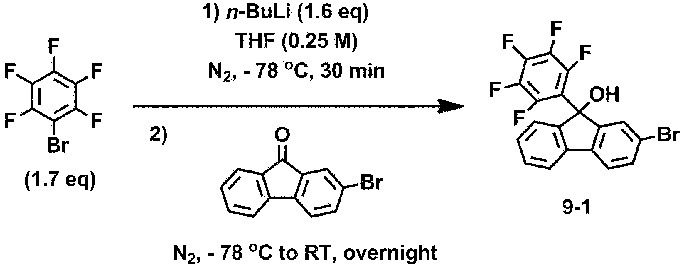

Intermediate 9-1 was prepared in the same manner as in (1) of Preparation Example 1, except that 1-bromo-2,3,4,5,6-pentafluorobenzene (31.8 mL, 255 mmol) was used instead of 1-bromo-4-fluorobenzene in (1) of Preparation Example 1.

### (2) Synthesis of Intermediate 9-2

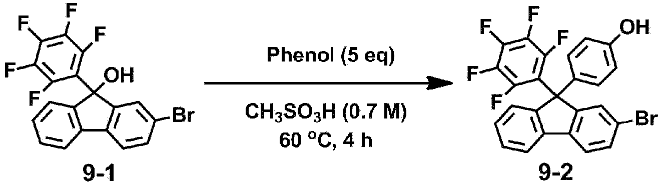

52.8 g of Intermediate 9-2 was obtained by preparation in the same manner as in (2) of Preparation Example 1, except that Intermediate 9-1 (64.1 g, 150 mmol) was used instead of Intermediate 1-1 in (2) of Preparation Example 1.

### (3) Synthesis of Intermediate 9-3

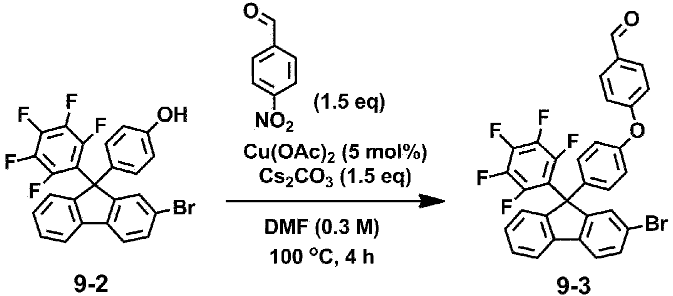

48.6 g of Intermediate 9-3 was obtained by preparation in the same manner as in (3) of Preparation Example 1, except that Intermediate 9-2 (50.3 g, 100 mmol) was used instead of Intermediate 1-2 in (3) of Preparation Example 1.

### (4) Synthesis of Intermediate 9-4

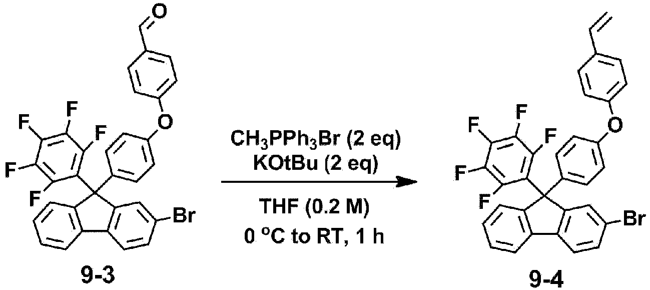

31 g of Intermediate 9-4 was obtained by preparation in the same manner as in (4) of Preparation Example 1, except that Intermediate 9-3 (43.7 g, 72 mmol) was used instead of Intermediate 1-3 in (4) of Preparation Example 1.

### (5) Synthesis of Compound 9

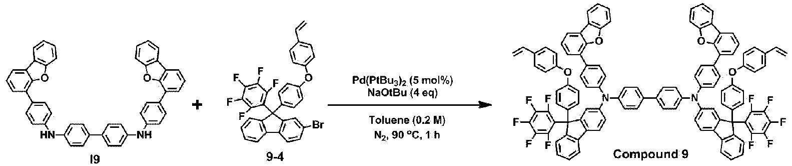

2.8 g of Compound 9 was obtained by preparation in the same manner as in (5) of Preparation Example 1, except that Compound I9 (1.67 g, 2.5 mmol) and Intermediate 9-4 (3.3 g, 5.5 mmol) were used instead of Compound I1 and Intermediate 1-4, respectively, in (5) of Preparation Example 1. It was confirmed through LC-MS and NMR that Compound 9 was synthesized. MS: [M+H]⁺ = 1717
NMR measurement value of Compound 9: ¹H NMR (500 MHz, DMSO-d6) δ 8.08 (m, 2H), 8.02 (m, 2H), 7.87 - 7.70 (m, 10H), 7.47 - 7.32 (m, 10H), 7.15 (m, 2H), 7.05 - 6.90 (m, 8H), 6.90 - 6.85 (m, 12H), 6.77 - 6.65 (m, 8H), 6.53 (d, 4H), 6.51 (m, 2H), 6.36 (m, 2H), 5.71 (dd, 2H), 5.18 (dd, 2H)

### <Device Example>

### Experimental Example 1.

A glass substrate thinly coated with indium tin oxide (ITO) to have a thickness of 1,500 Å was put into distilled water in which a detergent was dissolved, and ultrasonically washed. In this case, a product manufactured by the Fischer Co., was used as the detergent, and distilled water twice filtered using a filter manufactured by Millipore Co., was used as the distilled water. After the ITO was washed for 30 minutes, ultrasonic washing was conducted twice repeatedly using distilled water for 10 minutes. After the washing with distilled water was completed, the substrate was ultrasonically washed with a solvent of isopropyl alcohol and acetone, and dried, and then the substrate was cleaned for 5 minutes, and the substrate was transported to a glovebox.

On an ITO transparent electrode, a 2 wt% cyclohexanone ink including Compound 1 previously prepared in Preparation Example 1 and the following Compound G at a weight ratio of 8:2 was spin-coated onto the ITO surface, and heat-treated at 220°C for 30 minutes, thereby forming a hole injection layer having a thickness of 400 Å.

A 2 wt% toluene ink of the following Compound A was spin-coated onto the hole injection layer, and the coated hole injection layer was heat-treated at 120°C for 10 minutes, thereby forming a hole transport layer having a thickness of 200 Å. The following Compound B and Compound C were vacuum-deposited at a weight ratio of 92:8 on the hole transport layer, thereby forming a light emitting layer having a thickness of 200 Å. The following Compound D was vacuum-deposited on the light emitting layer, thereby forming an electron transport and injection layer having a thickness of 350 Å. LiF and aluminum were sequentially deposited on the electron transport and injection layer to have a thickness of 10 Å and 1000 Å, respectively, thereby forming a cathode.

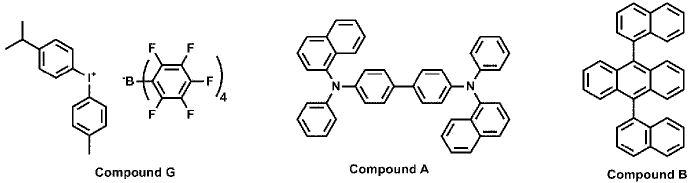

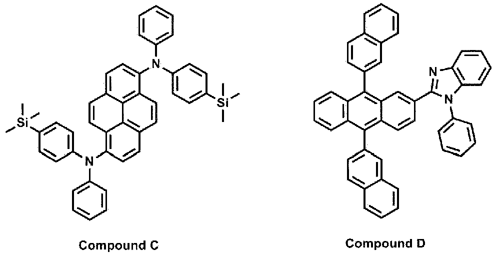

In the aforementioned procedure, the deposition rate of the organic material was maintained at 0.4 to 0.7 Å/sec, the deposition rates of lithium fluoride and aluminum of the cathode were maintained at 0.3 Å/sec and at 2 Å/sec, respectively, and the degree of vacuum during the deposition was maintained at 2 × 10⁻⁷ torr to 5 × 10⁻⁸ torr.

### Experimental Examples 2 to 9.

Organic light emitting devices were manufactured in the same manner as in Experimental Example 1, except that the compounds described in the following Table 1 were used instead of Compound 1 during the manufacture of the hole injection layer (HIL).

### Comparative Examples 1 to 15

Organic light emitting devices were manufactured in the same manner as in Experimental Example 1, except that the compounds described in the following Table 1 were used instead of Compound 1 during the manufacture of the hole injection layer.

Compounds CE1 to CE15 used in Comparative Examples 1 to 15 are as follows.

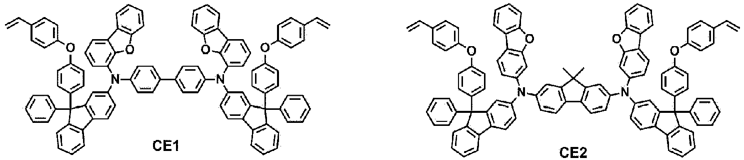

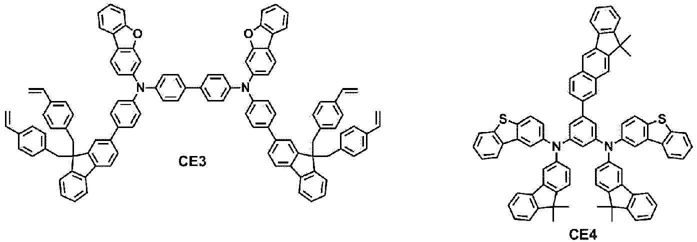

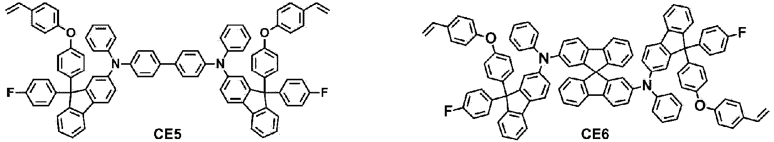

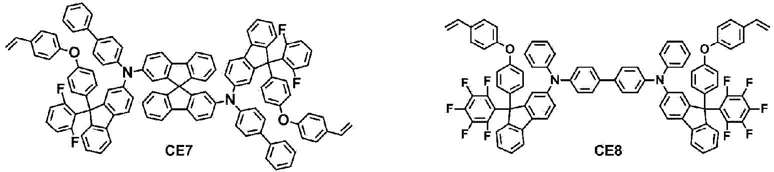

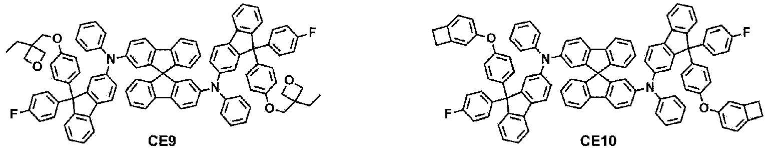

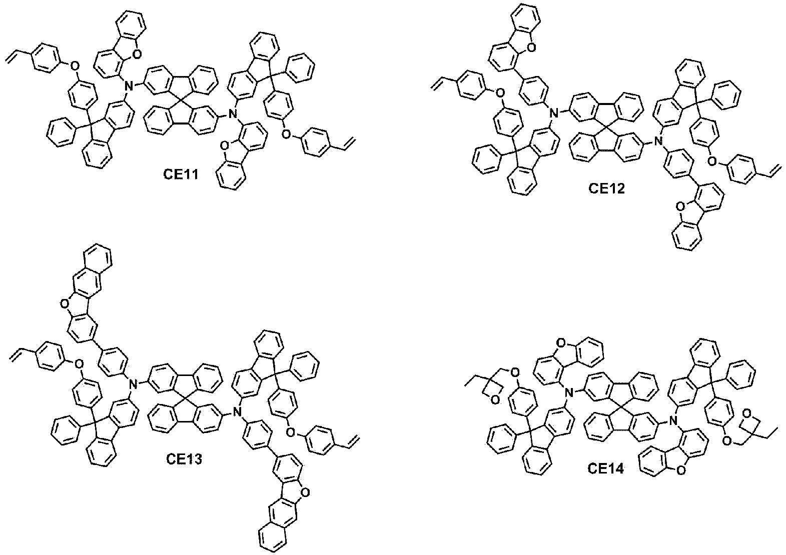

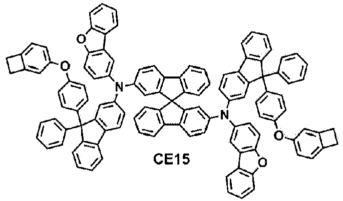

For the organic light emitting device manufactured in each of the Experimental Examples and Comparative Examples, the results of measuring the driving voltage, external quantum efficiency, luminance and service life at a current density of 10 mA/cm² are shown in the following Table 1. The external quantum efficiency was obtained by (the number of emitted photons)/(the number of injected charge carriers). T95 means the time (hr) taken for the luminance to be reduced to 95% of the initial luminance (500 nit).

**[Table 1]**

| | HIL host | Driving voltage (V) | External quantum efficiency (%) | Luminance (cd/m² ) | T95 (hr) @500 nit |
|---|---|---|---|---|---|
| Experimental Example 1 | Compound 1 | 4.71 | 5.6 | 597 | 193 |
| Experimental Example 2 | Compound 2 | 4.53 | 5.5 | 603 | 191 |
| Experimental Example 3 | Compound 3 | 4.55 | 5.6 | 610 | 153 |
| Experimental Example 4 | Compound 4 | 4.49 | 5.8 | 624 | 188 |
| Experimental Example 5 | Compound 5 | 4.86 | 5.1 | 547 | 159 |
| Experimental Example 6 | Compound 6 | 4.73 | 5.3 | 573 | 182 |
| Experimental Example 7 | Compound 7 | 4.74 | 5.4 | 585 | 151 |
| Experimental Example 8 | Compound 8 | 4.90 | 5.2 | 553 | 164 |
| Experimental Example 9 | Compound 9 | 4.81 | 5.2 | 561 | 175 |
| Comparative Example 1 | CE1 | 5.47 | 4.9 | 462 | 92 |
| Comparative Example 2 | CE2 | 5.51 | 4.7 | 425 | 88 |
| Comparative Example 3 | CE3 | 6.13 | 5.2 | 514 | 51 |
| Comparative Example 4 | CE4 | 8.50 | 1.3 | 100 | 30 |
| Comparative Example 5 | CE5 | 5.22 | 4.7 | 428 | 134 |
| Comparative Example 6 | CE6 | 5.31 | 4.9 | 459 | 141 |
| Comparative Example 7 | CE7 | 5.28 | 4.6 | 420 | 137 |
| Comparative Example 8 | CE8 | 5.38 | 4.8 | 447 | 121 |
| Comparative Example 9 | CE9 | 5.56 | 4.7 | 441 | 127 |
| Comparative Example 10 | CE10 | 5.63 | 4.1 | 395 | 115 |
| Comparative Example 11 | CE11 | 5.47 | 5.0 | 465 | 101 |
| Comparative Example 12 | CE12 | 5.50 | 5.1 | 522 | 123 |
| Comparative Example 13 | CE13 | 5.61 | 4.9 | 476 | 115 |
| Comparative Example 14 | CE14 | 5.32 | 4.7 | 441 | 102 |
| Comparative Example 15 | CE15 | 5.44 | 4.1 | 395 | 95 |

In Experimental Examples 1 to 9, the compound represented by Chemical Formula 1 according to the present invention is used as the host of the hole injection layer, and in Comparative Examples 1 to 15, compounds CE1, CE2, and CE11 to CE15 (Comparative Examples 1, 2, and 11 to 15) to which no fluoro group (-F) is bonded, a compound CE3 (Comparative Example 3) including 4 or more curable groups, Compound CE4 (Comparative Example 4) including no curable group or compounds CE5 to CE10 (Comparative Examples 5 to 10) including an aryl group instead of a heteroaryl group are used.

As shown in Table 1, it can be confirmed that the driving voltage is significantly reduced and the service life is significantly improved in the organic light emitting devices (Experimental Examples 1 to 9) in which the compound represented by Chemical Formula 1 according to the present invention is used as a host of a hole injection layer, compared to the organic light emitting devices of Comparative Examples 1 to 15. Further, it can be confirmed that the efficiency and luminance are increased in the organic light emitting devices of Experimental Examples 1 to 9 compared to the organic light emitting devices of Comparative Examples 1 to 15. Through this, it could be confirmed that film and interfacial characteristics were improved by a heteroaryl group (a dibenzofuran group, and the like) and a fluoro group (-F) introduced into the molecule, and the change in HOMO level improves hole mobility and hole/electron balance, thereby improving the performance of the organic light emitting device. Meanwhile, it is considered that in the case of Comparative Example 3 in which a compound having 4 or more curable groups was used, the driving voltage is increased and the service life is decreased by the curable group which is not cured and remains even after the heat treatment. Since Comparative Example 4, in which the compound including no curable group is used, does not have solvent resistance even after heat treatment, it can be seen that the device does not properly function because most of the materials are washed away during the film-formation of the hole transport layer (HTL).

## Claims

1. A compound represented by the following Chemical Formula 1: 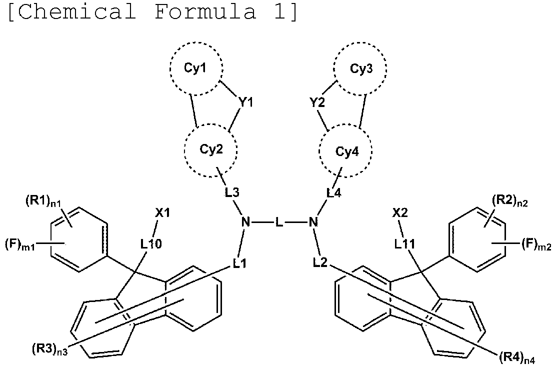 wherein, in Chemical Formula 1,
Cy1 to Cy4 are the same as or different from each other, and are each independently a substituted or unsubstituted hydrocarbon ring group,
Y1 and Y2 are the same as or different from each other, and are each independently O, S, Se or NR,
L is a substituted or unsubstituted divalent hydrocarbon ring group; or a substituted or unsubstituted divalent heterocyclic group,
L1 to L4 are the same as or different from each other, and are each independently a direct bond; a substituted or unsubstituted arylene group; or a substituted or unsubstituted heteroarylene group,
L10 and L11 are the same as or different from each other, and are each independently a substituted or unsubstituted arylene group,
X1 and X2 are the same as or different from each other, and are each independently a curable group,
R and R1 to R4 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
n1 and n2 are each an integer from 0 to 4, and when n1 and n2 are each 2 or higher, two or more substituents in the parenthesis are the same as or different from each other,
m1 and m2 are each an integer from 1 to 5, n1+m1 is 5 or lower, and n2+m2 is 5 or lower, and
n3 and n4 are each an integer from 1 to 7, and when n3 and n4 are each 2 or higher, two or more substituents in the parenthesis are the same as or different from each other.

2. The compound of claim 1, wherein the curable group is any one of the following structures: 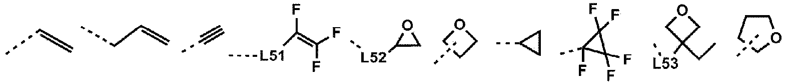 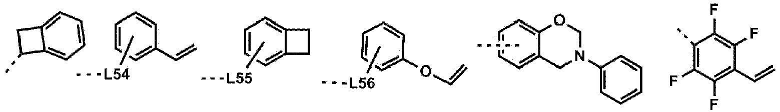 in the structures,
L51 to L56 are the same as or different from each other, and are each independently any one or two or more linked groups selected from the group consisting of a direct bond; -O-; a substituted or unsubstituted alkylene group; and a substituted or unsubstituted arylene group, and
- - - - - - is a moiety bonded to Chemical Formula 1.

3. The compound of claim 1, wherein Chemical Formula 1 is the following Chemical Formula 2: 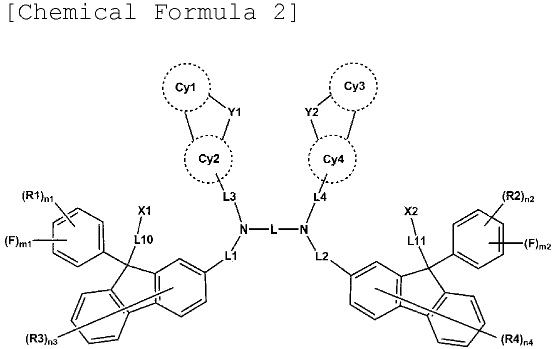 in Chemical Formula 2,
the definitions of Cy1 to Cy4, Y1, Y2, L, L1 to L4, L10, L11, X1, X2, R1 to R4, n1 to n4, m1 and m2 are the same as those in Chemical Formula 1.

4. The compound of claim 1, wherein Chemical Formula 1 is the following Chemical Formula 3: 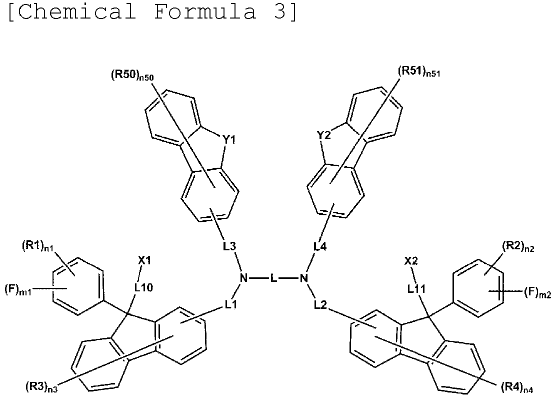 in Chemical Formula 3,
the definitions of Y1, Y2, L, L1 to L4, L10, L11, X1, X2, R1 to R4, n1 to n4, m1 and m2 are the same as those in Chemical Formula 1,
R50 and R51 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group, or are bonded to an adjacent group to form a substituted or unsubstituted ring, and
n50 and n51 are each an integer from 1 to 7, and when n50 and n51 are each 2 or higher, two or more substituents in the parenthesis are the same as or different from each other.

5. The compound of claim 1, wherein L is any one of the following structures: 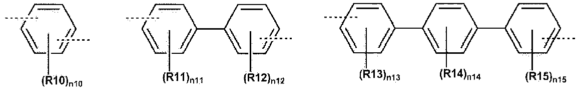 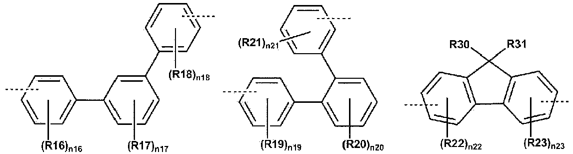 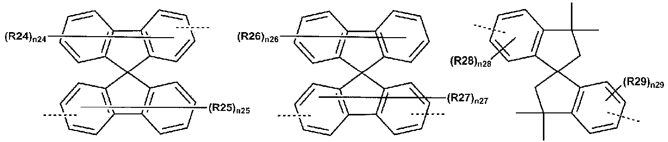 in the structures,
R10 to R31 are the same as or different from each other, and are each independently hydrogen; deuterium; a halogen group; a substituted or unsubstituted alkyl group; a substituted or unsubstituted alkoxy group; a substituted or unsubstituted aryl group; or a substituted or unsubstituted heteroaryl group,
n10 to n21 are each an integer from 1 to 4, n22, n23, n28 and n29 are each an integer from 1 to 3, n24 and n25 are each an integer from 1 to 7, n26 is an integer from 1 to 8, n27 is an integer from 1 to 6, and when n10 to n29 are each 2 or higher, two or more substituents in the parenthesis are the same as or different from each other, and
------ is a moiety bonded to Chemical Formula 1.

6. The compound of claim 1, wherein L1 to L4 are the same as or different from each other, and are each independently a direct bond; or a substituted or unsubstituted C₆₋₃₀ arylene group.

7. The compound of claim 1, wherein R1 to R4 are the same as or different from each other, and are each independently hydrogen; deuterium; or a substituted or unsubstituted C₁₋₃₀ alkyl group.

8. The compound of claim 1, wherein L10 and L11 are the same as or different from each other, and are each independently a substituted or unsubstituted phenylene group; a substituted or unsubstituted biphenylene group; a substituted or unsubstituted terphenylene group; or a substituted or unsubstituted naphthylene group.

9. The compound of claim 1, wherein the compound of Chemical Formula 1 is any one of the following structures:

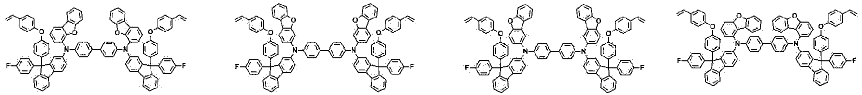

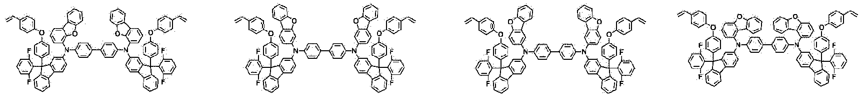

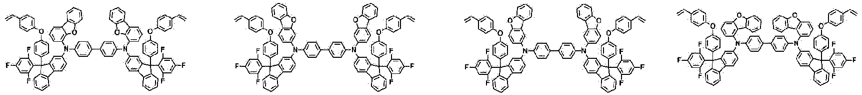

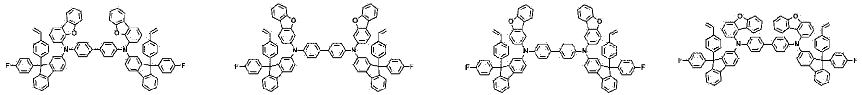

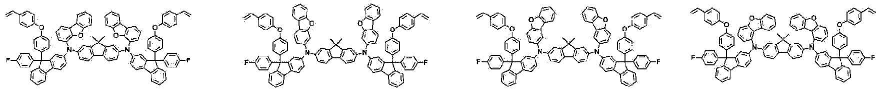

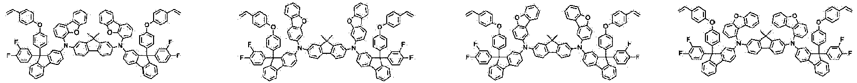

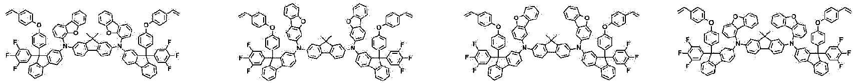

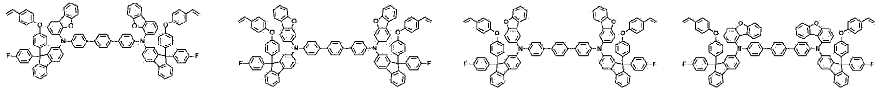

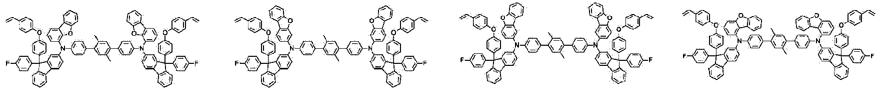

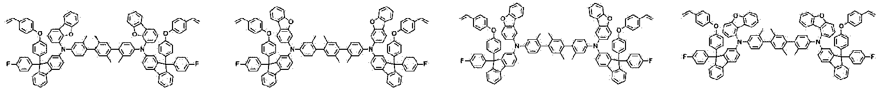

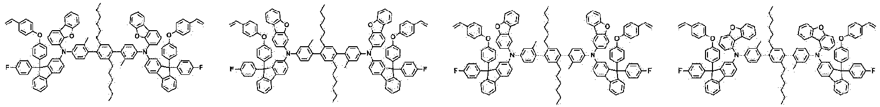

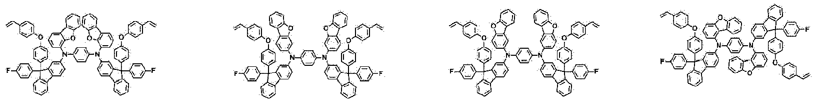

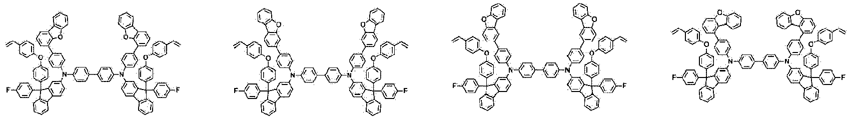

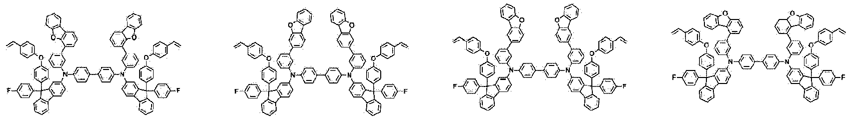

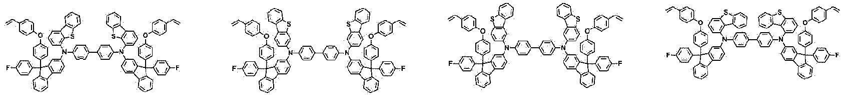

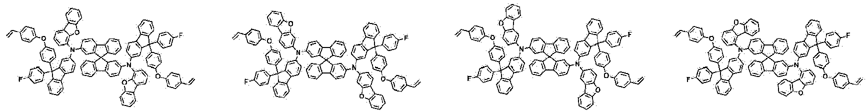

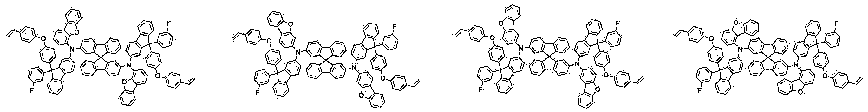

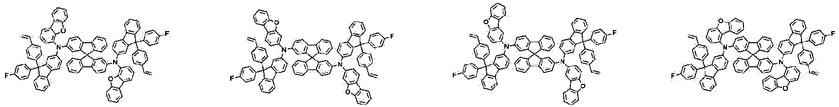

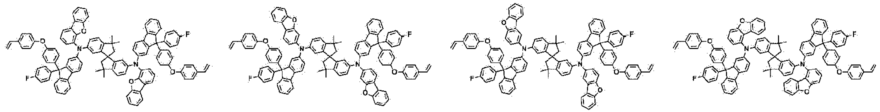

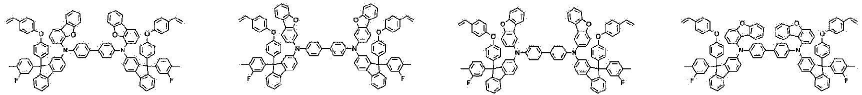

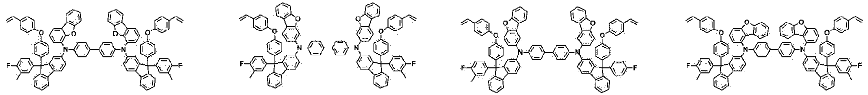

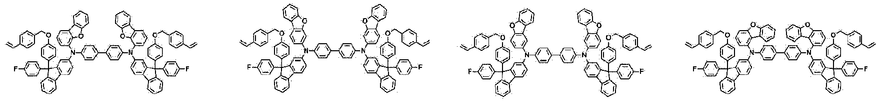

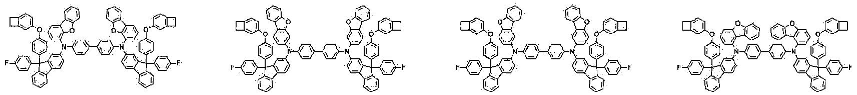

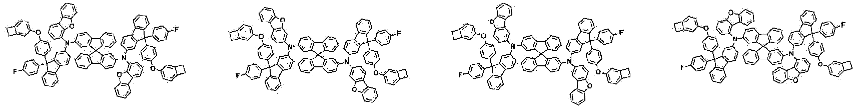

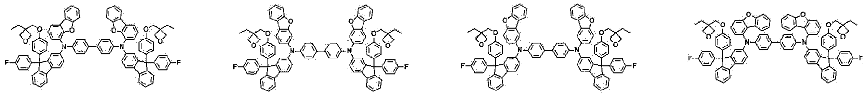

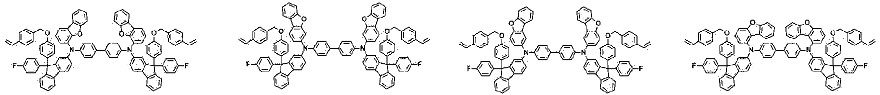

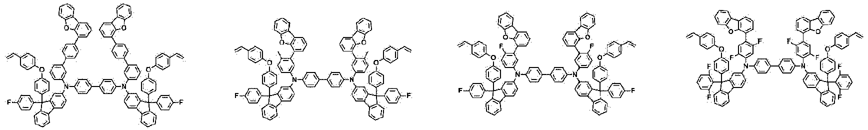

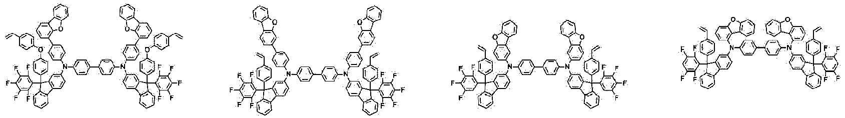

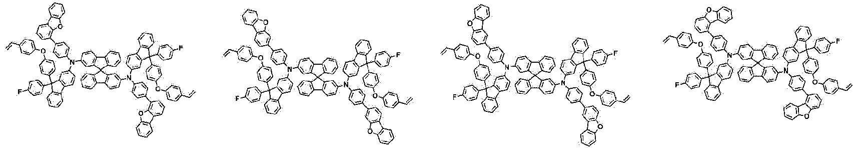

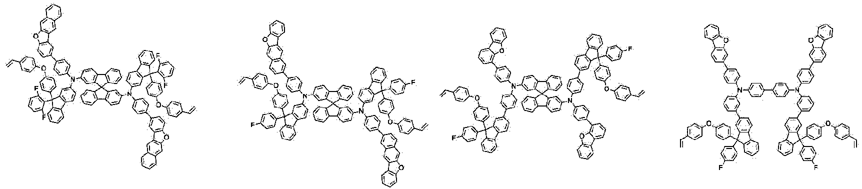

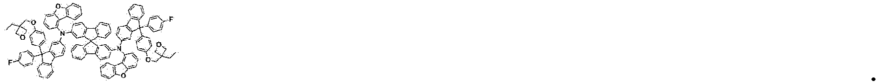

10. A coating composition comprising the compound of any one of claims 1 to 9.

11. An organic light emitting device comprising: a first electrode;
a second electrode; and
an organic material layer having one or more layers provided between the first electrode and the second electrode,
wherein one or more layers of the organic material layer comprise the coating composition of claim 10 or a cured product thereof.

12. The organic light emitting device of claim 11, wherein the organic material layer comprising the coating composition or the cured product thereof is a hole transport layer, a hole injection layer, or a layer which simultaneously transports and injects holes.

13. A method for manufacturing an organic light emitting device, the method comprising: preparing a first electrode;
forming an organic material layer having one or more layers on the first electrode; and
forming a second electrode on the organic material layer,
wherein the forming of the organic material layer comprises forming an organic material layer having one or more layers using the coating composition of claim 10.

14. The method of claim 13, wherein the forming of the organic material layer using the coating composition comprises:
coating the coating composition; and
heat-treating or light-treating the coated coating composition.

## Patentansprüche

1. Verbindung, dargestellt durch die folgende chemische Formel 1: 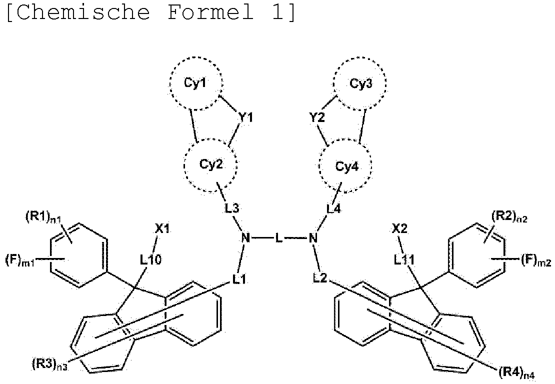 worin in der chemischen Formel 1,
Cy1 bis Cy4 gleich oder verschieden sind und jeweils unabhängig eine substituierte oder unsubstituierte Kohlenwasserstoffringgruppe sind,
Y1 und Y2 gleich oder verschieden sind und jeweils unabhängig O, S, Se oder NR sind,
L eine substituierte oder unsubstituierte zweiwertige Kohlenwasserstoffringgruppe oder eine substituierte oder unsubstituierte zweiwertige heterocyclische Gruppe ist,
L1 bis L4 gleich oder verschieden sind und jeweils unabhängig eine direkte Bindung; eine substituierte oder unsubstituierte Arylengruppe; oder eine substituierte oder unsubstituierte Heteroarylengruppe sind,
L10 und L11 gleich oder verschieden sind und jeweils unabhängig eine substituierte oder unsubstituierte Arylengruppe sind,
X1 und X2 gleich oder verschieden sind und jeweils unabhängig eine härtbare Gruppe sind,
R und R1 bis R4 gleich oder verschieden sind und jeweils unabhängig Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte Heteroarylgruppe sind,
n1 und n2 jeweils eine ganze Zahl von 0 bis 4 sind und wenn n1 und n2 jeweils 2 oder höher sind, zwei oder mehr Substituenten in der Klammer gleich oder verschieden sind,
m1 und m2 jeweils eine ganze Zahl von 1 bis 5 sind, n1+m1 5 oder weniger ist und n2+m2 5 oder weniger ist, und
n3 und n4 jeweils eine ganze Zahl von 1 bis 7 sind, und wenn n3 und n4 jeweils 2 oder höher sind, zwei oder mehr Substituenten in der Klammer gleich oder verschieden sind.

2. Verbindung gemäß Anspruch 1, worin die härtbare Gruppe eine der folgenden Strukturen aufweist: 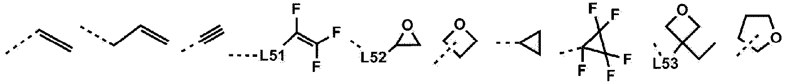 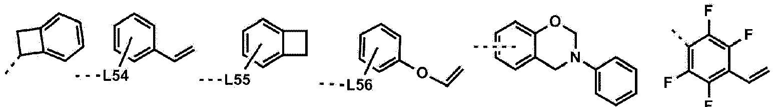 worin in den Strukturen
L51 bis L56 gleich oder verschieden sind und jeweils unabhängig eine oder zwei oder mehr verbundene Gruppen sind, die ausgewählt sind aus der Gruppe bestehend aus einer direkten Bindung; -O-; einer substituierten oder unsubstituierten Alkylengruppe; und einer substituierten oder unsubstituierten Arylengruppe, und
------ eine an die chemische Formel 1 gebundene Einheit ist.

3. Verbindung gemäß Anspruch 1, wobei die chemische Formel 1 die folgende chemische Formel 2 ist: 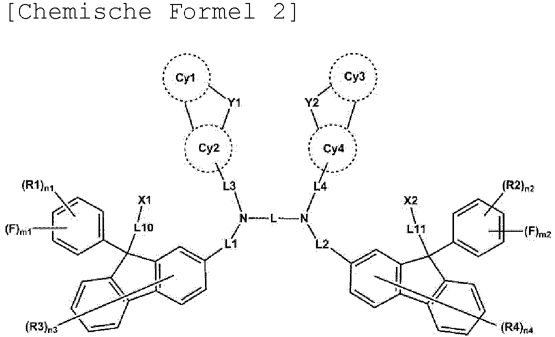 worin in der chemischen Formel 2,
die Definitionen von Cy1 bis Cy4, Y1, Y2, L, L1 bis L4, L10, L11, X1, X2, R1 bis R4, n1 bis n4, m1 und m2 die gleichen sind wie in der chemischen Formel 1.

4. Verbindung gemäß Anspruch 1, wobei die chemische Formel 1 die folgende chemische Formel 3 ist: 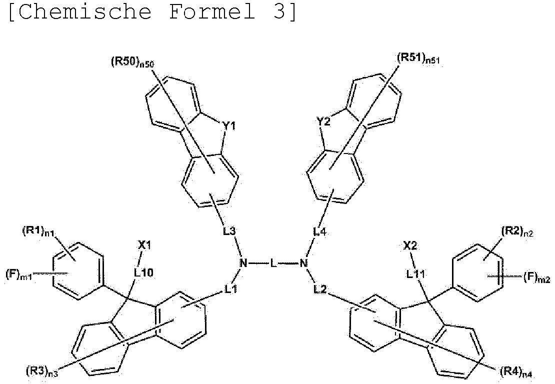 worin in der chemischen Formel 3,
die Definitionen von Y1, Y2, L, L1 bis L4, L10, L11, X1, X2, R1 bis R4, n1 bis n4, m1 und m2 die gleichen sind wie in der chemischen Formel 1,
R50 und R51 gleich oder verschieden sind und jeweils unabhängig Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte Heteroarylgruppe sind, oder an eine benachbarte Gruppe gebunden sind, um einen substituierten oder unsubstituierten Ring zu bilden, und
n50 und n51 jeweils eine ganze Zahl von 1 bis 7 sind, und wenn n50 und n51 jeweils 2 oder höher sind, zwei oder mehr Substituenten in der Klammer gleich oder verschieden sind.

5. Verbindung gemäß Anspruch 1, worin L eine der folgenden Strukturen aufweist: 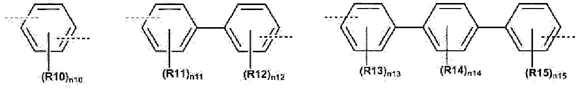 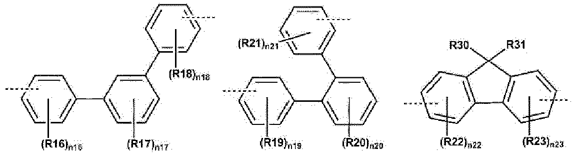 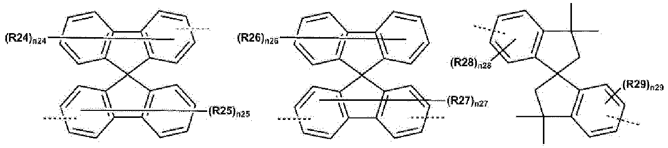 worin in den Strukturen,
R10 bis R31 gleich oder verschieden sind und jeweils unabhängig Wasserstoff; Deuterium; eine Halogengruppe; eine substituierte oder unsubstituierte Alkylgruppe; eine substituierte oder unsubstituierte Alkoxygruppe; eine substituierte oder unsubstituierte Arylgruppe; oder eine substituierte oder unsubstituierte Heteroarylgruppe sind;
n10 bis n21 jeweils eine ganze Zahl von 1 bis 4 sind, n22, n23, n28 und n29 jeweils eine ganze Zahl von 1 bis 3 sind, n24 und n25 jeweils eine ganze Zahl von 1 bis 7 sind, n26 eine ganze Zahl von 1 bis 8 ist, n27 eine ganze Zahl von 1 bis 6 ist, und wenn n10 bis n29 jeweils 2 oder höher sind, zwei oder mehr Substituenten in der Klammer gleich oder verschieden sind, und
------ eine an die chemische Formel 1 gebundene Einheit ist.

6. Verbindung gemäß Anspruch 1, worin L1 bis L4 gleich oder verschieden sind und jeweils unabhängig eine direkte Bindung oder eine substituierte oder unsubstituierte C₆₋₃₀-Arylengruppe sind.

7. Verbindung gemäß Anspruch 1, worin R1 bis R4 gleich oder verschieden sind und jeweils unabhängig Wasserstoff; Deuterium; oder eine substituierte oder unsubstituierte C₁₋₃₀-Alkylgruppe sind.

8. Verbindung gemäß Anspruch 1, worin L10 und L11 gleich oder verschieden sind und jeweils unabhängig eine substituierte oder unsubstituierte Phenylengruppe; eine substituierte oder unsubstituierte Biphenylengruppe; eine substituierte oder unsubstituierte Terphenylengruppe; oder eine substituierte oder unsubstituierte Naphthylengruppe sind.

9. Verbindung gemäß Anspruch 1, wobei die Verbindung der chemischen Formel 1 eine der folgenden Strukturen ist:

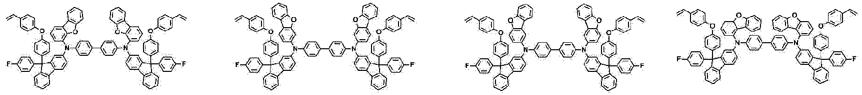

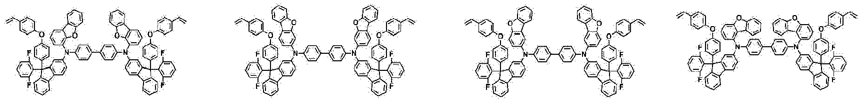

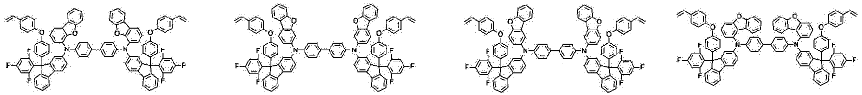

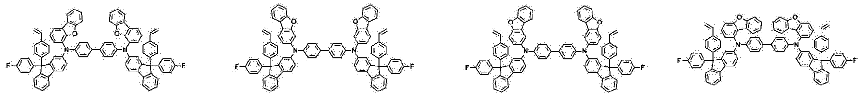

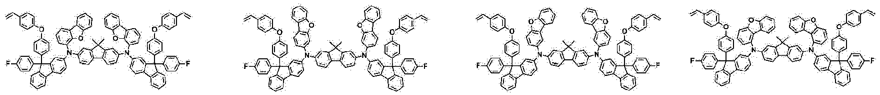

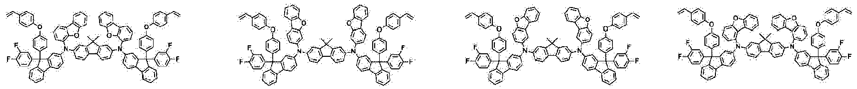

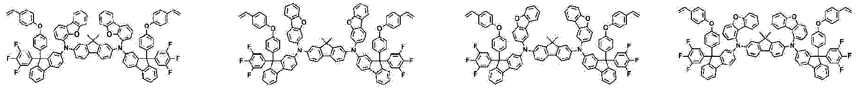

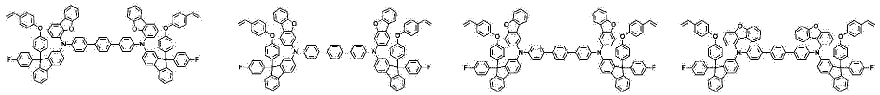

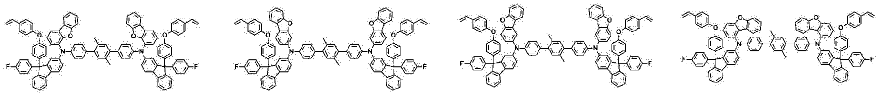

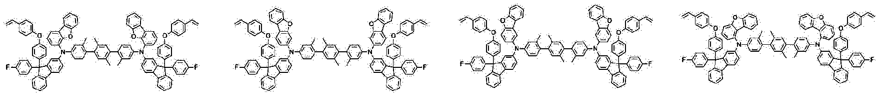

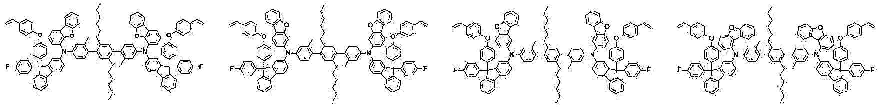

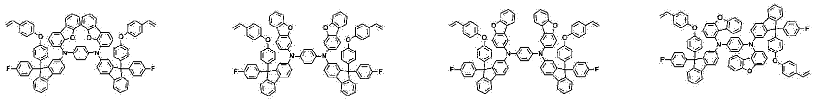

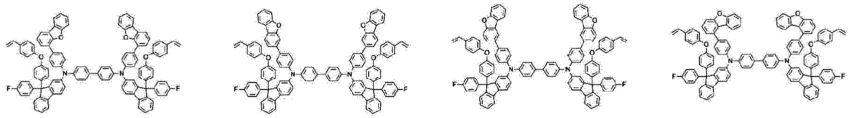

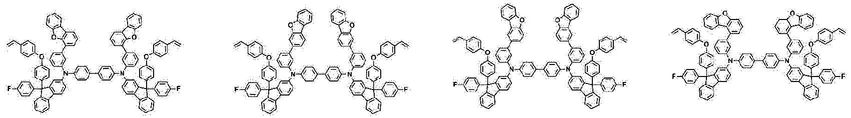

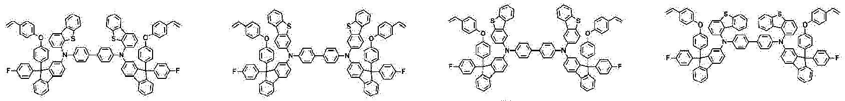

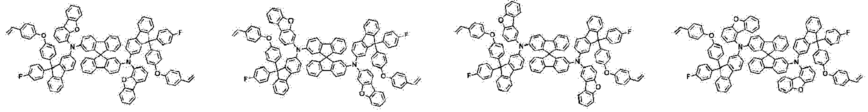

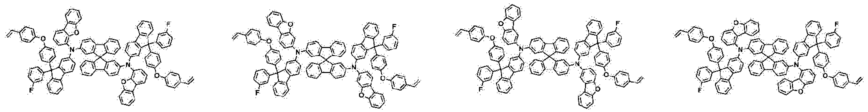

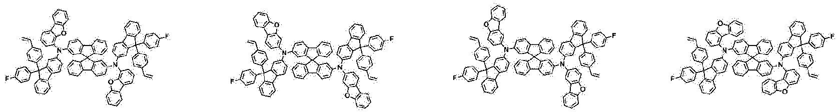

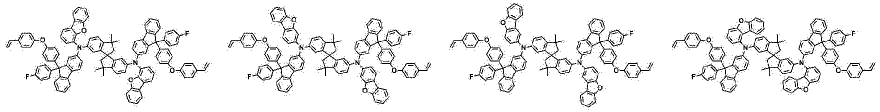

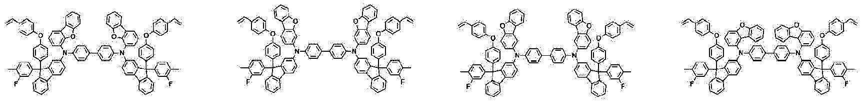

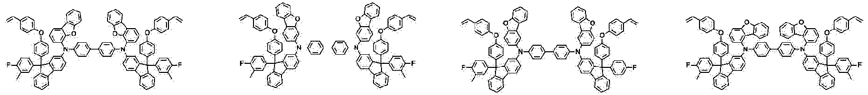

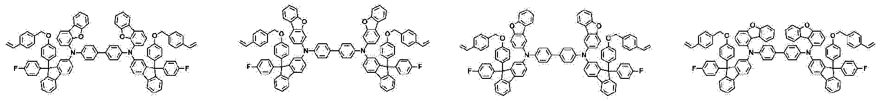

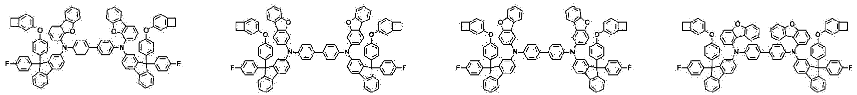

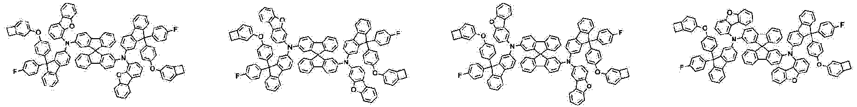

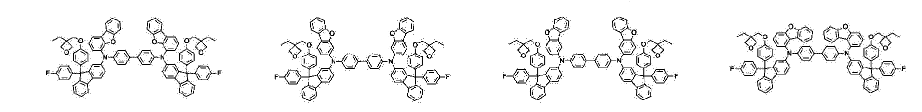

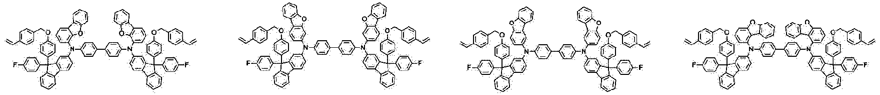

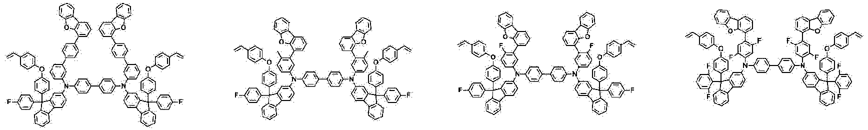

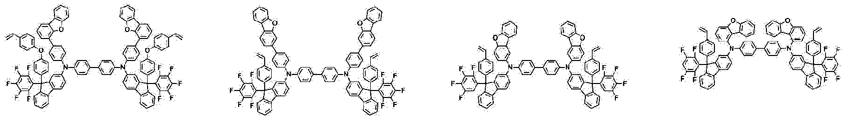

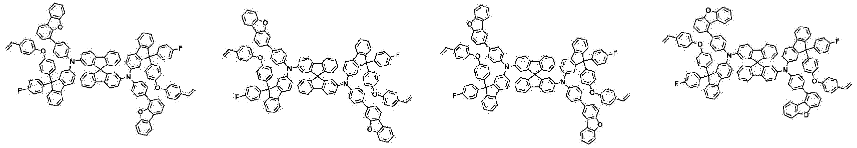

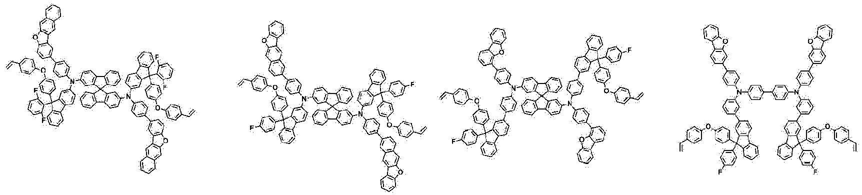

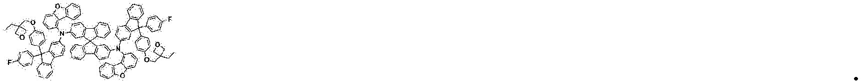

10. Beschichtungszusammensetzung, umfassend die Verbindung gemäß mindestens einem der Ansprüche 1 bis 9.

11. Organische lichtemittierende Vorrichtung, umfassend:
eine erste Elektrode;
eine zweite Elektrode; und
eine Schicht aus organischem Material mit einer oder mehreren Schichten, die zwischen der ersten Elektrode und der zweiten Elektrode vorgesehen sind,
wobei eine oder mehrere Schichten der Schicht aus organischem Material die Beschichtungszusammensetzung gemäß Anspruch 10 oder ein ausgehärtetes Produkt davon umfassen.

12. Organische lichtemittierende Vorrichtung gemäß Anspruch 11, wobei die Schicht aus organischem Material, die die Beschichtungszusammensetzung oder das ausgehärtete Produkt davon umfasst, eine Lochtransportschicht, eine Lochinjektionsschicht oder eine Schicht ist, die gleichzeitig Löcher transportiert und injiziert.

13. Verfahren zur Herstellung einer organischen lichtemittierenden Vorrichtung, wobei das Verfahren umfasst:
Herstellen einer ersten Elektrode;
Bilden einer Schicht aus organischem Material mit einer oder mehreren Schichten auf der ersten Elektrode; und
Bilden einer zweiten Elektrode auf der Schicht aus organischem Material,
wobei das Bilden der Schicht aus organischem Material das Bilden einer Schicht aus organischem Material mit einer oder mehreren Schichten unter Verwendung der Beschichtungszusammensetzung gemäß Anspruch 10 umfasst.

14. Verfahren gemäß Anspruch 13, wobei das Bilden der Schicht aus organischem Material unter Verwendung der Beschichtungszusammensetzung umfasst:
Auftragen der Beschichtungszusammensetzung; und
Wärmebehandeln oder Lichtbehandeln der aufgetragenen Beschichtungszusammensetzung.

## Revendications

1. Composé représenté par la formule chimique 1 suivante : 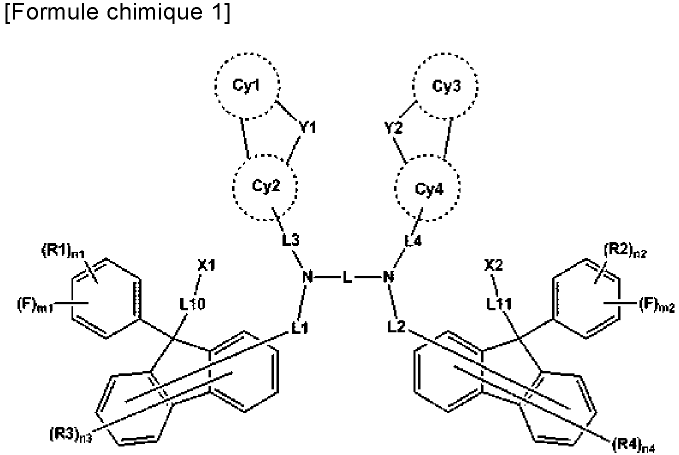 dans lequel, dans la formule chimique 1,
Cy1 à Cy4 sont identiques les uns aux autres ou différents les uns des autres, et sont chacun indépendamment un groupe anneau hydrocarboné substitué ou non substitué,
Y1 et Y2 sont identiques l'un à l'autre ou différents l'un de l'autre, et sont chacun indépendamment 0, S, Se ou NR,
L est un groupe anneau hydrocarboné divalent substitué ou non substitué ; ou un groupe hétérocyclique divalent substitué ou non substitué,
L1 à L4 sont identiques les uns aux autres ou différents les uns des autres, et sont chacun indépendamment une liaison directe ; un groupe arylène substitué ou non substitué ; ou un groupe hétéroarylène substitué ou non substitué ;
L10 et L11 sont identiques l'un à l'autre ou différents l'un de l'autre, et sont chacun indépendamment un groupe arylène substitué ou non substitué,
X1 et X2 sont identiques l'un à l'autre ou différents l'un de l'autre, et sont chacun indépendamment un groupe durcissable ;
R et R1 à R4 sont identiques les uns aux autres ou différents les uns des autres, et sont chacun indépendamment hydrogène ; deutérium ; un groupe halogène ; un groupe alkyle substitué ou non substitué ; un groupe alcoxy substitué ou non substitué ; un groupe aryle substitué ou non substitué ; ou un groupe hétéroaryle substitué ou non substitué,
n1 et n2 sont chacun un entier de 0 à 4, et lorsque n1 et n2 sont chacun 2 ou plus, deux substituants ou plus entre parenthèses sont identiques l'un à l'autre ou différents l'un de l'autre,
m1 et m2 sont chacun un entier de 1 à 5, n1+m1 est 5 ou moins, et n2+m2 est 5 ou moins, et
n3 et n4 sont chacun un entier de 1 à 7, et lorsque n3 et n4 sont chacun 2 ou plus, deux substituants ou plus entre parenthèses sont identiques l'un à l'autre ou différents l'un de l'autre.

2. Composé selon la revendication 1, dans lequel le groupe durcissable est l'une quelconque des structures suivantes : 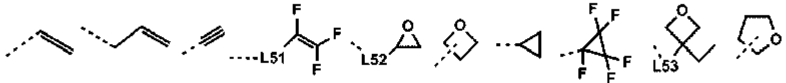 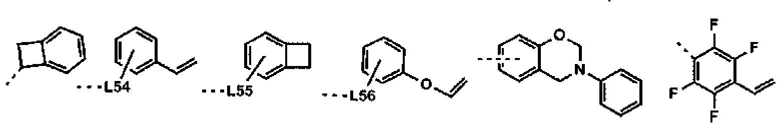 dans les structures,
L51 à L56 sont identiques les uns aux autres ou différents les uns des autres, et sont chacun indépendamment un ou deux groupes liés ou plus choisis dans le groupe consistant en une liaison directe ; -o- ; un groupe alkylène substitué ou non substitué ; et un groupe arylène substitué ou non substitué, et
-------- est une fraction liée à la formule chimique 1.

3. Composé selon la revendication 1, dans lequel la formule chimique 1 est la formule chimique 2 suivante : 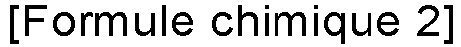 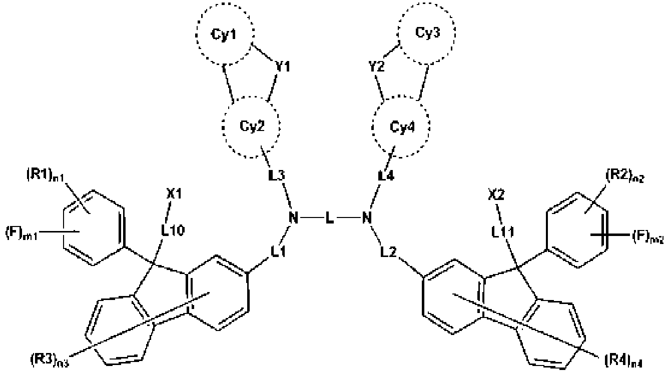 dans la formule chimique 2,
les définitions de Cy1 à Cy4, Y1, Y2, L, L1 à L4, L10, L11, X1, X2, R1 à R4, n1 à n4, m1 et m2 sont les mêmes que celles de la formule chimique 1.

4. Composé selon la revendication 1, dans lequel la formule chimique 1 est la formule chimique 3 suivante : 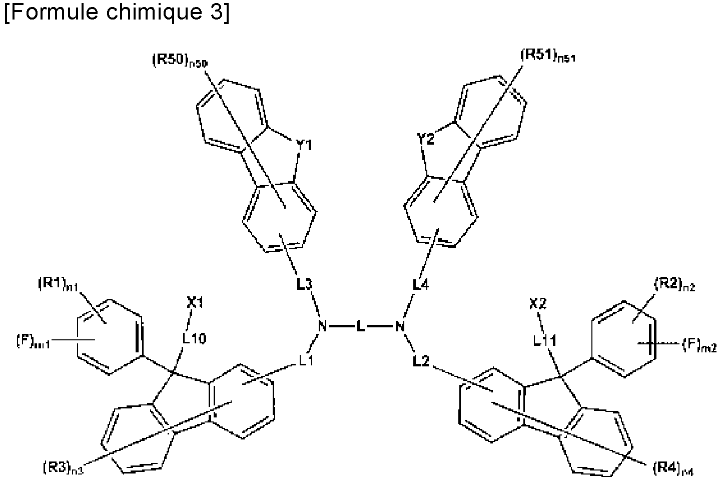 dans la formule chimique 3,
les définitions de Y1, Y2, L, L1 à L4, L10, L11, X1, X2, R1 à R4, n1 à n4, m1 et m2 sont les mêmes que celles de la formule chimique 1,
R50 et R51 sont identiques l'un à l'autre ou différents l'un de l'autre, et sont chacun indépendamment hydrogène ; deutérium ; un groupe halogène ; un groupe alkyle substitué ou non substitué ; un groupe alcoxy substitué ou non substitué ; un groupe aryle substitué ou non substitué ; ou un groupe hétéroaryle substitué ou non substitué, ou sont liés à un groupe adjacent pour former un anneau substitué ou non substitué, et
n50 et n51 sont chacun un entier de 1 à 7, et lorsque n50 et n51 sont chacun 2 ou plus, deux substituants ou plus entre parenthèses sont identiques l'un à l'autre ou différents l'un de l'autre.

5. Composé selon la revendication 1, dans lequel L est l'une quelconque des structures suivantes : 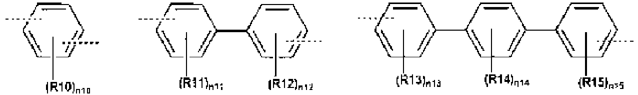 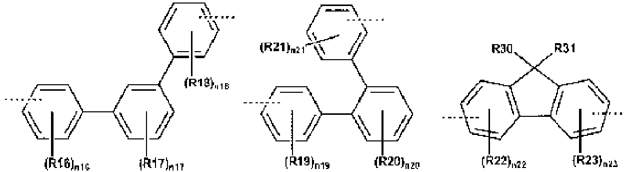 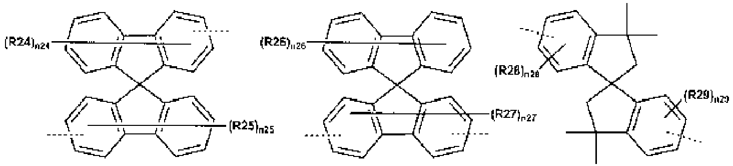 dans les structures,
R10 à R31 sont identiques les uns aux autres ou différents les uns des autres, et sont chacun indépendamment hydrogène ; deutérium ; un groupe halogène ; un groupe alkyle substitué ou non substitué ; un groupe alcoxy substitué ou non substitué ; un groupe aryle substitué ou non substitué ; ou un groupe hétéroaryle substitué ou non substitué,
n10 à n21 sont chacun un entier de 1 à 4, n22, n23, n28 et n29 sont chacun un entier de 1 à 3, n24 et n25 sont chacun un entier de 1 à 7, n26 est un entier de 1 à 8, n27 est un entier de 1 à 6, et lorsque n10 à n29 sont chacun 2 ou plus, deux substituants ou plus entre parenthèses sont identiques l'un à l'autre ou différents l'un de l'autre, et
-------- est une fraction liée à la formule chimique 1.

6. Composé selon la revendication 1, dans lequel L1 à L4 sont identiques les uns aux autres ou différents les uns des autres, et sont chacun indépendamment une liaison directe ; ou un groupe arylène C₆₋₃₀ substitué ou non substitué.

7. Composé selon la revendication 1, dans lequel R1 à R4 sont identiques les uns aux autres ou différents les uns des autres, et sont chacun indépendamment hydrogène ; deutérium ; ou un groupe alkyle C₁₋₃₀ substitué ou non substitué.

8. Composé selon la revendication 1, dans lequel L10 et L11 sont identiques l'un à l'autre ou différents l'un de l'autre, et sont chacun indépendamment un groupe phénylène substitué ou non substitué ; un groupe biphénylène substitué ou non substitué ; un groupe terphénylène substitué ou non substitué ; ou un groupe naphtylène substitué ou non substitué.

9. Composé selon la revendication 1, dans lequel le composé de la formule chimique 1 est l'une quelconque des structures suivantes :

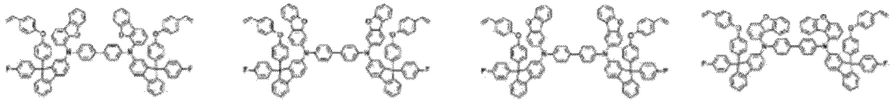

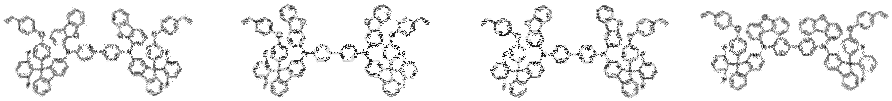

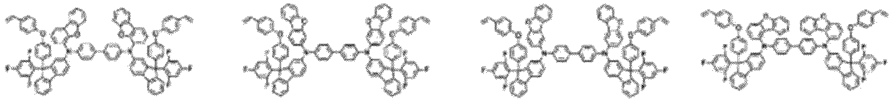

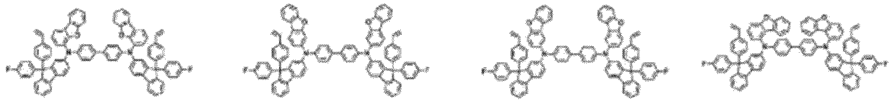

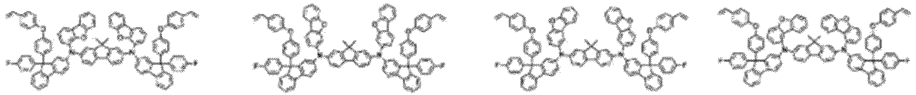

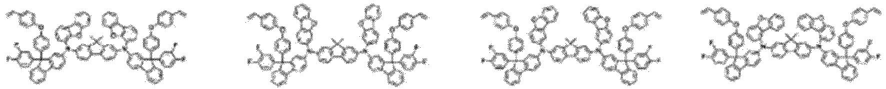

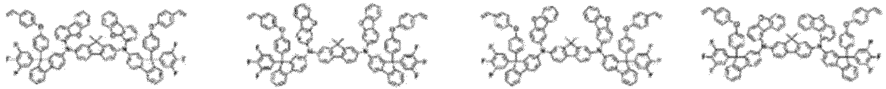

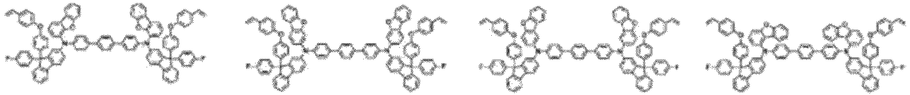

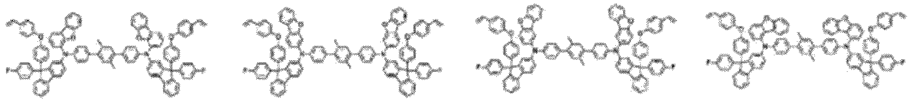

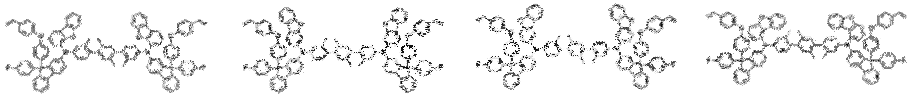

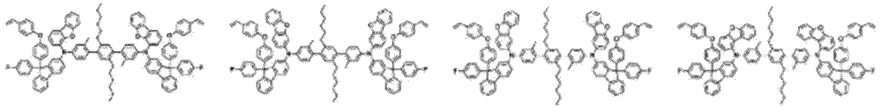

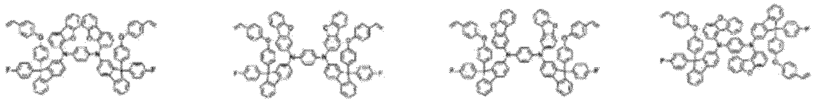

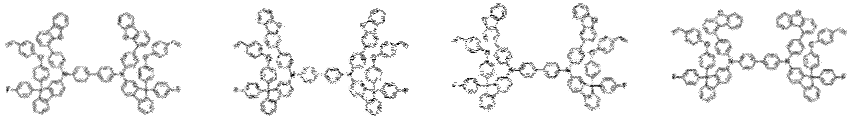

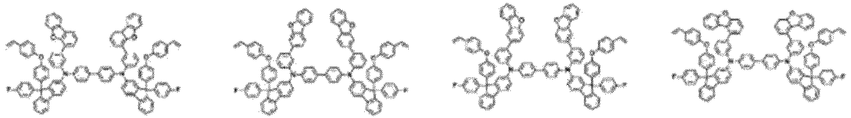

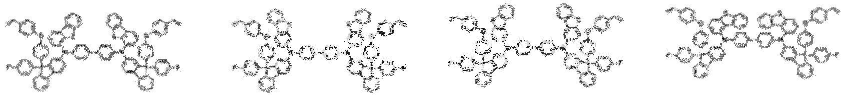

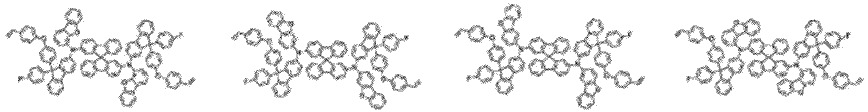

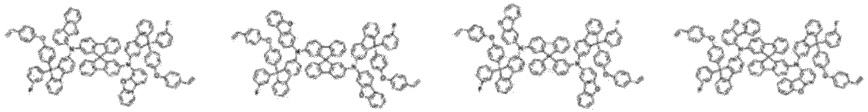

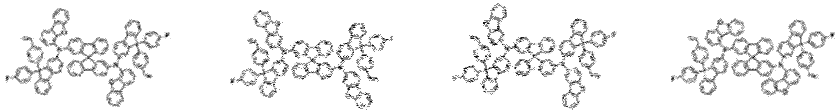

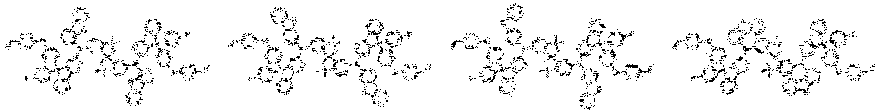

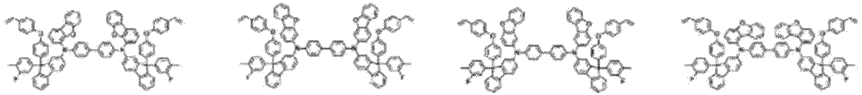

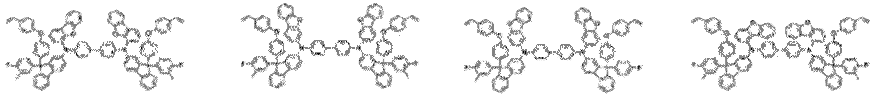

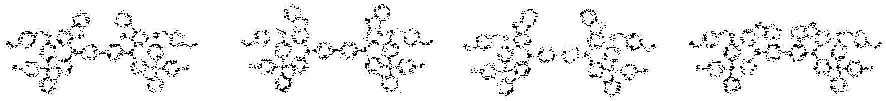

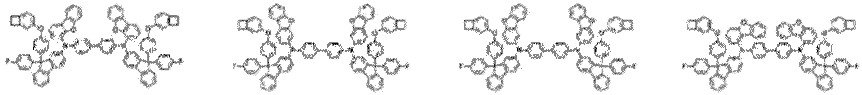

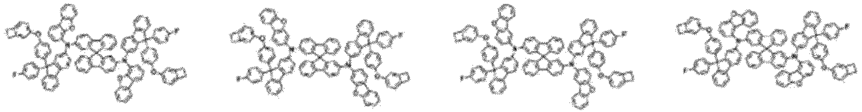

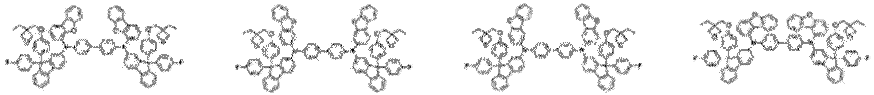

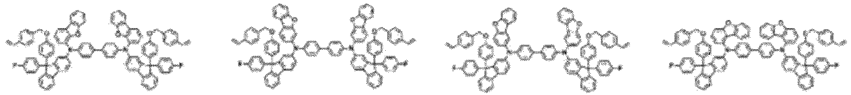

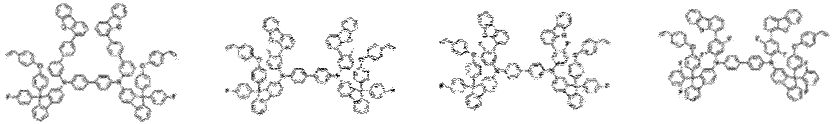

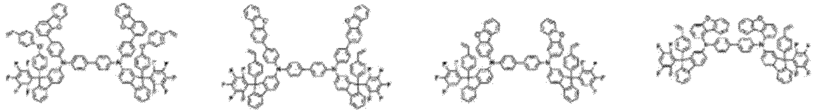

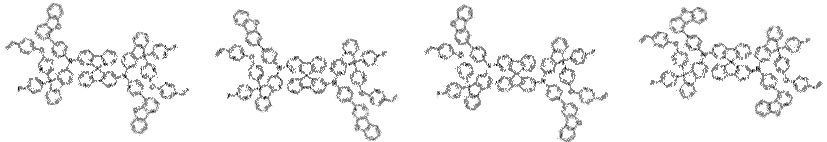

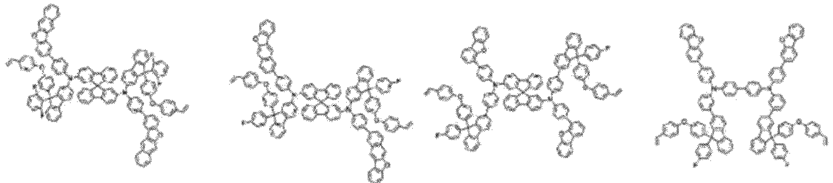

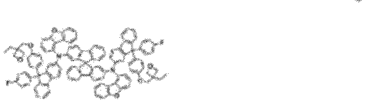

10. Composition de revêtement comprenant le composé selon l'une quelconque des revendications 1 à 9.

11. Dispositif électroluminescent organique comprenant : une première électrode ;
une seconde électrode ; et
une couche de matériau organique présentant une ou plusieurs couches disposées entre la première électrode et la seconde électrode,
dans lequel les une ou plusieurs couches de matériau organique comprennent la composition de revêtement selon la revendication 10 ou un produit durci de celle-ci.

12. Dispositif électroluminescent organique selon la revendication 11, dans lequel la couche de matériau organique comprenant la composition de revêtement ou le produit durci de celle-ci est une couche de transport de trous, une couche d'injection de trous, ou une couche qui transporte et injecte simultanément des trous.

13. Procédé pour fabriquer un dispositif électroluminescent organique, le procédé comprenant : la préparation d'une première électrode ;
la formation d'une couche de matériau organique présentant une ou plusieurs couches sur la première électrode ; et
la formation d'une seconde électrode sur la couche de matériau organique,
dans lequel la formation de la couche de matériau organique comprend la formation d'une couche de matériau organique présentant une ou plusieurs couches en utilisant la composition de revêtement selon la revendication 10.

14. Procédé selon la revendication 13, dans lequel la formation de la couche de matériau organique en utilisant la composition de revêtement comprend :
le revêtement de la composition de revêtement ; et
le traitement thermique ou traitement lumineux de la composition de revêtement revêtue.
